# EUROPÄISCHE PATENTSCHRIFT

(11) **EP 1 280 787 B1**
(45) Veröffentlichungstag und Bekanntmachung des Hinweises auf die Patenterteilung: **02.01.2008**
(21) Anmeldenummer: 01951476.9
(22) Anmeldetag: 03.05.2001
(51) Int. Cl.: C07D 307/08, C07D 307/33, C07C 29/80

(54) **VERFAHREN ZUR DESTILLATIVEN TRENNUNG VON TETRAHYDROFURAN, GAMMA-BUTYROLACTON UND/ODER 1,4-BUTANDIOL ENTHALTENDEN GEMISCHEN**
METHOD FOR DISTILLATIVE SEPARATION OF MIXTURES CONTAINING TETRAHYDROFURAN, GAMMA-BUTYROLACTONE AND/OR 1,4-BUTANEDIOL
PROCEDE POUR LA SEPARATION PAR DISTILLATION DE MELANGES CONTENANT DU TETRAHYDROFURANE, DE LA GAMMA-BUTYROLACTONE ET/OU DU 1,4-BUTANEDIOL

(30) Priorität: 04.05.2000 DE 10021703
(43) Veröffentlichungstag der Anmeldung: 05.02.2003
(73) Patentinhaber: BASF Aktiengesellschaft, 67056 Ludwigshafen (DE)
(72) Erfinder: KAIBEL, Gerd, 68623 Lampertheim (DE); WECK, Alexander, 67251 Freinsheim (DE); RAHN, Ralf-Thomas, 68167 Mannheim (DE)
(74) Vertreter: Pohl, Michael Friedrich
(86) Internationale Anmeldenummer: PCT/EP2001/004974
(87) Internationale Veröffentlichungsnummer: WO 2001/085708

(56) Entgegenhaltungen:
- EP-A- 0 255 400
- EP-A- 0 301 852
- WO-A-91/01981
- DE-A- 3 726 805
- DE-A- 3 938 121
- US-A- 4 919 765
- US-A- 5 310 954

## Beschreibung

Die vorliegende Erfindung betrifft ein kontinuierlich betriebenes Verfahren zur destillativen Trennung von Tetrahydrofuran, γ-Butyrolacton und/oder 1,4-Butandiol enthaltenden Gemischen in mindestens drei Fraktionen.

Bei der technischen Herstellung von 1,4-Butandiol, Tetrahydrofuran und γ-Butyrolacton bedient man sich im verstärkten Maße Kohlenwasserstoffen mit mindestens vier Kohlenstoffatomen als wirtschaftliche Rohstoffquelle. Wichtige Einsatzstoffe sind Benzol, n-Butene und n-Butan. In einer ersten Stufe werden die Kohlenwasserstoffe heterogenkatalytisch in der Gasphase zu Maleinsäureanhydrid oxidiert. Eine Übersicht gängiger Verfahren ist gegeben in Ullmann's Encyclopedia of Industrial Chemistry, 6th Edition, 1999 Electronic Release, Chapter "Maleic and Fumaric Acids, Maleic Anhydride". Das gebildete Maleinsäureanhydrid wird durch Abkühlen aus dem Gasstrom abgeschieden oder in Wasser oder organischen Absorptionsmitteln absorbiert. Je nach Wahl der Aufarbeitung werden daraus Maleinsäureanhydrid, Maleinsäure, deren Ester, deren Halbester oder Gemische daraus erhalten. Diese Verbindungen werden anschließend als solche oder nach einer gezielten Umwandlung zu Maleinsäureanhydrid, Maleinsäure, deren Ester, deren Halbester oder Gemische in der Flüssig- oder Gasphase heterogenkatalytisch hydriert. Derartige heterogenkatalytische Hydrierungen sind beispielsweise in der Offenlegungsschrift EP-A 0 304 696 für Maleinsäureanhydrid, Bernsteinsäureanhydrid oder deren Säuren, Ester oder Halbester in der Flüssigphase, in WO 97/24346 für Maleinsäureanhydrid, Bernsteinsäureanhydrid oder deren Säuren in der Gasphase, in WO 97/43242 für Maleinsäuredialkylester in der Gasphase und in WO 97/43234 für Maleinsäureanhydrid in der Gasphase beschrieben. Je nach eingesetztem Katalysator und eingestellten Reaktionsbedingungen werden Hydrierprodukte erhalten, welche Tetrahydrofuran, γ-Butyrolacton und/oder 1,4-Butandiol enthalten.

Während 1,4-Butandiol ein wichtiges Zwischenprodukt bei der Herstellung von Polyurethanen, Polybutylenterephthalaten und Tetrahydrofuran ist, wird Tetrahydrofuran hauptsächlich zur Herstellung von Polytetrahydrofuran eingesetzt. Zudem dient es als vielseitiges Lösungsmittel, beispielsweise für Harze und Polyvinylchlorid. γ-Butyrolacton ist wichtiger Ausgangsstoff für zahlreiche Synthesen, wie beispielsweise die Herstellung von N-Methylpyrrolidon, Pyrrolidon, Polyvinylpyrrolidon und Herbiziden und wird als vielseitiges Lösungsmittel für Polymere eingesetzt.

Für ihre weitere Verwendung müssen Tetrahydrofuran, γ-Butyrolacton und 1,4-Butandiol in sehr reiner Form gewonnen werden. Dies trifft insbesondere für Tetrahydrofuran zu, wenn es zu Polytetrahydrofuran polymerisiert und anschließend zu Fasern versponnen werden soll. Als Standardoperation für die Reinigungsschritte hat sich die destillative Aufarbeitung durchgesetzt. Diese wird nach dem Stand der Technik durch mehrere Destillationskolonnen realisiert, welche, für die Zerlegung von Mehrstoffgemischen, in konventioneller Weise verschaltet sind.

Für die kontinuierliche destillative Zerlegung von Mehrstoffgemischen sind verschiedene Verfahrensvarianten gebräuchlich. Im einfachsten Fall wird das Zulaufgemisch in eine leichtsiedende Kopffraktion und eine schwersiedende Sumpffraktion getrennt. Bei der Auftrennung von Zulaufgemischen in mehr als 2 Fraktionen müssen nach dieser Verfahrensvariante mehrere Destillationskolonnen eingesetzt werden. Um den apparativen Aufwand zu begrenzen, setzt man bei der Auftrennung von Vielstoffgemischen nach Möglichkeit Kolonnen mit flüssigen oder dampfförmigen Seitenabzügen ein. Nachteilig ist jedoch, da die an den Seitenabzugsstellen entnommenen Produkte nie völlig rein sind. Die Verwendung von konventionellen Seitenabzugskolonnen ist daher auf Fälle begrenzt, in denen verunreinigte Seitenprodukte zulässig sind oder durch zusätzliche, nachgeschaltete Kolonnen nachdestilliert werden.

So werden nach CHEM SYSTEMS, Band 91S15, "Butanediol/Tetrahydrofuran", April 1993, Seite 54-59 zur Aufarbeitung der Gemische, welche bei der Hydrierung von Maleinsäurediethylester anfallen, insgesamt sechs Destillationskolonnen eingesetzt. In der ersten Kolonne wird Tetrahydrofuran/Wasser-Gemisch über Kopf abgezogen und in einer zweiten Kolonne zu Tetrahydrofuran und Wasser getrennt. Das Sumpfprodukt der ersten Kolonne wird zur dritten Kolonne geführt und dort über Kopf Ethanol abgezogen. In der vierten Kolonne werden über Kopf weitere Nebenprodukte, in der fünften Kolonne γ-Butyrolacton und Bernsteinsäure-diethylester und in der sechsten Kolonne schließlich 1,4-Butandiol und Schwersieder voneinander getrennt.

Für die Abtrennung von Tetrahydrofuran aus einer Mischung enthaltend Wasser, Tetrahydrofuran und Alkohole hat sich eine sogenannte "Zwei-Druck-Destillation" bewährt, die in der Offenlegungsschrift WO 91/01981 beschrieben ist. In der ersten Kolonne wird dabei über Kopf eine Wasser, Tetrahydrofuran und Alkohole enthaltende Phase abgezogen und der zweiten Kolonne zugeführt.

Die zweite Kolonne wird bei einem höheren Druck als die erste betrieben und trennt über Sumpf reines Tetrahydrofuran ab. Die über Kopf abgezogene Phase wird in die erste Kolonne zurückgeführt.

Eine Aufgabe der vorliegenden Erfindung bestand darin, ein Verfahren zur kontinuierlich betriebenen, destillativen Trennung von Tetrahydrofuran, γ-Butyrolacton und/oder 1,4-Butandiol enthaltenden Gemischen zu finden, wobei mindestens eine Fraktion Tetrahydrofuran, γ-Butyrolacton und/oder 1,4-Butandiol enthält, und welches die Gewinnung der Wertprodukte in hoher Reinheit bei gleichzeitig geringem Energieverbrauch und geringen Investitionskosten der Anlage ermöglicht. Eine weitere Aufgabe der vorliegenden Erfindung war es, eine flexible und zugleich einfache Prozeßführung zu finden, welche auch bei Schwankungen in der Menge der Produktströme und deren Zusammensetzung einen sicheren Betrieb des Verfahrens unter Einhaltung der hohen Reinheitsanforderungen und eines geringen Energieverbrauchs gewährleistet.

Demgemäß wurde ein Verfahren zur kontinuierlich betriebenen, destillativen Trennung von Tetrahydrofuran, γ-Butyrolacton und/oder 1,4-Butandiol enthaltenden Gemischen in mindestens drei Fraktionen gefunden, das dadurch gekennzeichnet ist, da man die Trennung in einer Anordnung von Destillationskolonnen, die mindestens eine Trennwandkolonne oder mindestens eine Anordnung von thermisch gekoppelten konventionellen Destillationskolonnen enthält, durchführt.

Unter einer Anordnung von Destillationskolonnen ist mindestens eine Trennwandkolonne oder sind mindestens zwei konventionelle Destillationskolonnen zu verstehen. Enthält die Anordnung mehr als eine Kolonne, so sind die Kolonnen über die Weiterleitung und/oder den wechselseitigen Austausch von Massenströmen (Produktströmen) und/oder Energieströmen miteinander verbunden. Destillationskolonnen sind Apparate mit mindestens einer Zulaufstelle und mindestens zwei Entnahmestellen, bei denen sich zwischen Verdampfer und Kondensator der sogenannte Rektifizierbereich befindet, in dem ein Teil des im Kondensator gebildeten Kondensats sich flüssig als Rücklauf im Gegenstrom zu den aus der Verdampfungseinrichtung aufsteigenden Dämpfen abwärts bewegt und welche somit eine destillative Trennung von Stoffgemischen ermöglichen.

Wesentlich beim erfindungsgemäßen Verfahren ist, da die Anordnung der Destillationskolonnen mindestens eine Trennwandkolonne und/oder mindestens eine Anordnung von thermisch gekoppelten konventionellen Destillationskolonnen enthält. Trennwandkolonnen sind spezielle Destillationskolonnen mit mindestens einer Zulaufstelle und mindestens drei Entnahmestellen, bei denen sich zwischen Verdampfer und Kondensator der sogenannte Rektifizierbereich befindet, in dem ein Teil des im Kondensator gebildeten Kondensats sich flüssig als Rücklauf im Gegenstrom zu den aus der Verdampfungseinrichtung aufsteigenden Dämpfen abwärts bewegt und welche in einem Teilbereich der Kolonne unterhalb und/oder oberhalb der Zulaufstelle mindestens eine in Längsrichtung wirkende Trenneinrichtung (Trennwand) zur Verhinderung einer Quervermischung von Flüssigkeits- und/oder Brüdenstrom (Dampfstrom) enthält und welche somit eine destillative Trennung von Stoffgemischen ermöglichen. Das Grundprinzip der Trennwandkolonnen ist seit langem bekannt und beispielsweise im Patent US 2,471,134, in der Patentanmeldung EP-A-0 122 367 oder in G. Kaibel, Chem. Eng. Technol. Vol. 10, 1987, Seite 92 bis 98 beschrieben.

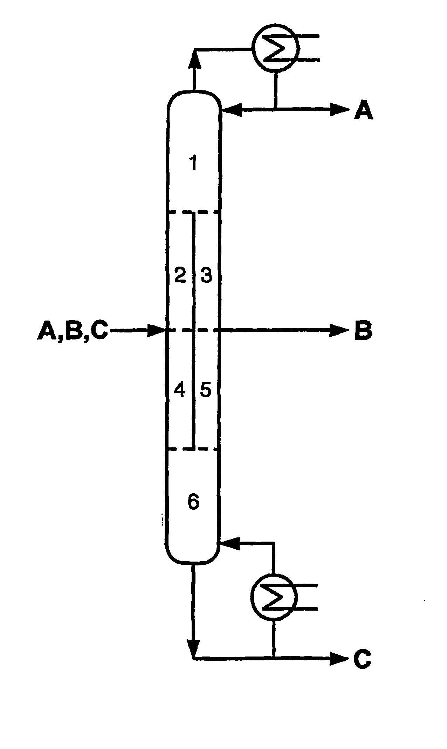

Fig.1 zeigt schematisch den allgemeinen Grundaufbau einer Trennwandkolonne. Sie besitzt mindestens eine seitliche Zulaufstelle diesseits der Trennwand und mindestens drei Entnahmestellen jenseits der Trennwand. Die Stoffströme sind wie folgt bezeichnet:
(A,B,C) Zulaufgemisch, welches an der Zulaufstelle zugeführt
(A) Leichtsiederfraktion, welche an der Kopfentnahmestelle entnommen wird,
(B) Mittelsiederfraktion, welche an der Seitenentnahmestelle entnommen wird,
(C) Schwersiederfraktion, welche an der Sumpfentnahmestelle entnommen wird.

Der Bereich innerhalb der Trennwandkolonne kann grob in die folgenden sechs Teilbereiche unterteilt werden:
(1) Kopf teil
(2) oberer Zulauf teil
(3) oberer Entnahmeteil
(4) unterer Zulaufteil
(5) unterer Entnahmeteil
(6) Sumpf teil.

Da bei dieser Bauart im Bereich der Trennwand eine Quervermischung von Flüssigkeits- und/oder Brüdenstrom verhindert wird, ist es möglich, auch Seitenprodukte in reiner Form zu erhalten. Hierdurch verringert sich bei der Auftrennung von Vielstoffgemischen im allgemeinen die Zahl der insgesamt benötigten Destillationskolonnen. Zudem können beim Einsatz von Trennwandkolonnen gegenüber einer einfachen Hintereinanderschaltung zweier konventioneller Destillationskolonnen Investitionskosten sowie Energie eingespart werden (siehe M. Knott, Process Engineering, Vol.2, 1993, February, Seite 33 bis 34). Als konventionelle Destillationskolonnen werden alle Destillationskolonnne bezeichnet, welche keine Trennwand enthalten.

Trennwandkolonnen können auch beim erfindungsgemäßen Verfahren durch eine Anordnung von thermisch gekoppelten konventionellen Destillationskolonnen ersetzt oder mit ihnen kombiniert werden. Bei thermisch gekoppelten konventionellen Destillationskolonnen werden Massen- und Energieströme wechselseitig ausgetauscht, so daß gegenüber einer einfachen Hintereinanderschaltung konventioneller Destillationskolonnen eine deutliche Einsparung von Energie möglich ist. Bevorzugt als Alternative zur Trennwandkolonne ist eine Verschaltung zweier thermisch gekoppelter Destillationskolonnen. Eine Übersicht verschiedener Anordnungen ist beispielsweise in G. Kaibel et al., Chem.-Ing.-Tech., Vol. 61, 1989, Seite 16 bis 25 und G. Kaibel et al., Gas Separation & Purification, Vol. 4, 1990, June, Seiten 109 bis 114 gegeben.

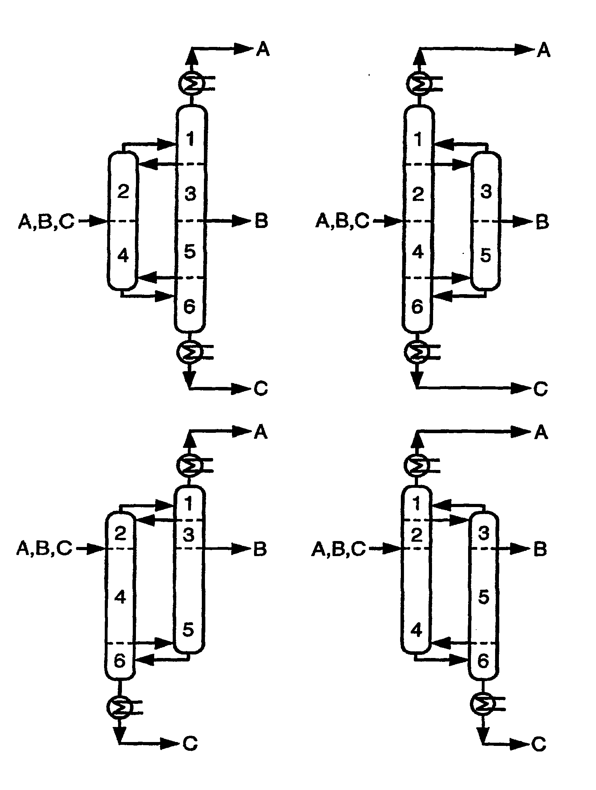

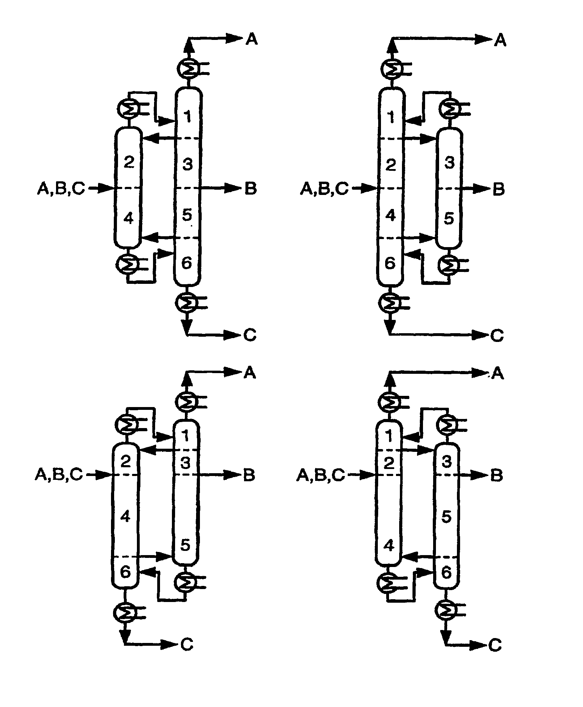

Fig. 2 und 3 zeigen, ohne limitierend zu wirken, schematisch Beispiele möglicher Anordnungen von jeweils zwei konventionellen Destillationskolonnen, welche einer Trennwandkolonne entsprechen. Bezüglich der Bezeichnungen (A,B,C), (A), (B), (C) und (1) bis (6) gilt die zuvor beschriebene Zuordnung. Beim erfindungsgemäßen Verfahren können Trennwandkolonnen prinzipiell durch die in Fig. 2 oder 3 schematisch dargestellten Anordnungen ersetzt werden.

Werden bereits vorhandene konventionelle Destillationskolonnen eingesetzt, wird in der Regel nach der Zahl der theoretischen Trennstufen der vorhandenen Kolonnen die geeignetste Form der Zusammenschaltung ausgewählt. Bereits vorhandene Apparate, wie etwa Verdampfer oder Kondensatoren, werden vorteilhafterweise gemäß den Anordnungen in Fig. 2 und 3 weiter genutzt. Es ist möglich, Schaltungsformen auszuwählen, die es erlauben, da nur flüssige Verbindungsströme zwischen den einzelnen Destillationskolonnen auftreten. Diese speziellen Verschaltungen bieten den Vorteil, da die beiden Destillationskolonnen unter verschiedenen Drücken betrieben werden können mit dem Vorteil, daß sie sich besser an die Temperaturniveaus vorhandener Heiz- und Kühlenergien anpassen lassen. Im allgemeinen wird man die Kolonne, an der die Leichtsiederfraktion entnommen wird im Druck höher wählen als die Kolonne, an der die Schwersiederfraktion entnommen wird.

Die Verwendung von zwei thermisch gekoppelten konventionellen Destillationskolonnen ist beispielsweise von Vorteil, wenn die einzelnen Kolonnen bereits vorhanden sind (z.B. bei Umbau, Ausbau, Erweiterung, Modernisierung einer bestehenden Anlage) oder wenn die beiden Kolonnen bei verschiedenen Drücken betrieben werden sollen.

Bei thermisch gekoppelten konventionellen Destillationskolonnen kann es von Vorteil sein, den Sumpfstrom der ersten Destillationskolonne in einem zusätzlichen Verdampfer teilweise oder vollständig zu verdampfen und danach der zweiten Destillationskolonne zuzuführen. Diese Vorverdampfung bietet sich insbesondere dann an, wenn der Sumpfstrom der ersten Destillationskolonne größere Mengen an Mittelsieder enthält. In diesem Fall kann die Vorverdampfung auf einem niedrigeren Temperaturniveau erfolgen und der Verdampfer der zweiten Destillationskolonne entlastet werden. Weiterhin wird durch diese Maßnahme der Abtriebsteil der zweiten Destillationskolonne wesentlich entlastet. Der vorverdampfte Strom kann dabei der zweiten Destillationskolonne zweiphasig oder in Form von zwei separaten Strömen zugeführt werden.

Darüber hinaus kann es sowohl bei Trennwandkolonnen als auch bei thermisch gekoppelten konventionellen Destillationskolonnen von Vorteil sein, den Zulaufstrom einer Vorverdampfung zu unterziehen und anschließend zweiphasig oder in Form von zwei Strömen der Destillationskolonne zuzuführen. Diese Vorverdampfung bietet sich besonders dann an, wenn der Zulaufstrom größere Mengen an Leichtsiedern enthält. Durch die Vorverdampfung kann der Abtriebsteil der Destillationskolonne wesentlich entlastet werden.

Beim erfindungsgemäßen Verfahren ist es möglich, zusätzlich zur erfindungsgemäßen Anordnung von Destillationskolonnen konventionelle Destillationskolonnen oder konventionelle Anordnungen von sogenannten Hauptkolonnen mit als Verstärkungs- oder Abtriebskolonne wirkenden Seitenkolonnen einzusetzen. Diese ermöglichen zwar ebenfalls die Gewinnung der Wertprodukte in hoher Reinheit, sind aber durch einen in der Regel höheren Energieverbrauch gekennzeichnet.

Da Trennwandkolonnen apparativ einfacher aufgebaut sind als thermisch gekoppelte konventionelle Destillationskolonnen, weisen diese in der Regel den Vorteil niedrigerer Investitionskosten.

Beim erfindungsgemäßen Verfahren ist daher die Verwendung von Trennwandkolonnen gegenüber thermisch gekoppelten konventionellen Destillationskolonnen bevorzugt, insbesondere beim Neubau von Anlagen.

Das beim erfindungsgemäßen Verfahren einzusetzende Gemisch enthält mindestens eine der Komponenten Tetrahydrofuran, γ-Butyrolacton oder 1,4-Butandiol. Die Zusammensetzung des Gemisches kann sehr unterschiedlich sein und ist stark von der Art und Weise des Herstellungsverfahrens abhängig. Im allgemeinen beträgt die Konzentration von Tetrahydrofuran 0 bis 70 Gew.-%, von γ-Butyrolacton 0 bis 85 Gew.-% und von 1,4-Butandiol 0 bis 85 Gew.-%. In der Regel enthält das Gemisch noch weitere Komponenten. Das Verfahren, durch das das Gemisch erhalten wurde, ist für den Erfolg des erfindungsgemäßen Verfahrens unkritisch.

Bevorzugt wird das Gemisch aus der Hydrierung von Maleinsäureanhydrid, Maleinsäure, deren Ester oder Halbester gewonnen. Das bevorzugte Gemisch kann zusätzlich das bei der Reaktion gebildete oder gegebenenfalls zugesetzte Wasser sowie Neben- und Koppelprodukte, wie beispielsweise Alkohole (z.B. Methanol, Ethanol, 1-Propanol, 2-Propanol, 1-Butanol, 2-Butanol, 2-Methyl-2-propanol, 2-Methyl-1-propanol aus den eingesetzten Estern), Bernsteinsäure, Bernsteinsäureanhydrid, Bernsteinsäureester, Buttersäure, Buttersäureester, Butadien, Methacrolein, Dihydrofurane (z.B. 2,3-Dihydrofuran) oder Glykolether (z.B. Dibutylenglykol) enthalten. Der Gehalt an Wasser liegt typischerweise bei 0 bis 35 Gew.-%.

Das beim erfindungsgemäßen Verfahren einzusetzende Tetrahydrofuran, γ-Butyrolacton und/oder 1,4-Butandiol enthaltende Gemisch wird besonders bevorzugt durch heterogenkatalytische Gasphasenhydrierung der oben genannten Komponenten erhalten, wie sie beispielsweise aus den Offenlegungsschriften WO 97/24346, WO 97/43242 und WO 97/43234 bekannt sind. Der bei der Hydrierung anfallende Produktstrom wird aus dem Hydrierreaktor geleitet und in der Regel heruntergekühlt. Beim bevorzugt eingesetzten-Gasphasenverfahren werden dabei die Wertprodukte, Wasser und der größte Anteil der Nebenprodukte auskondensiert. Nicht-umgesetzter Wasserstoff, Inertgase (z.B. Stickstoff und Edelgase) sowie sehr niedrig siedende Nebenprodukte verbleiben in der Gasphase und werden abgetrennt. Das so erhaltene flüssige Gemisch wird nun der erfindungsgemäßen, kontinuierlich betriebenen, destillativen Trennung zugeführt.

Beim erfindungsgemäßen Verfahren werden die Wertprodukte Tetrahydrofuran, γ-Butyrolacton und/oder 1,4-Butandiol abgetrennt und in hoher Reinheit gewonnen. Unter einer hohen Reinheit ist eine Konzentration von > 99,8 Gew.-% beim Tetrahydrofuran, > 99,5 Gew.-% beim γ-Butyrolacton und > 99,3 Gew.-% beim 1,4-Butandiol zu verstehen. Je nach gewünschter Ausführungsform des Verfahrens ist es möglich, alle drei Wertprodukte, eine Auswahl von zwei Wertprodukten oder auch nur eines der genannten Wertprodukte abzutrennen und in der gewünschten Reinheit zu erhalten.

Die destillative Trennung erfolgt beim erfindungsgemäßen Verfahren in mindestens drei Fraktionen. Unter Fraktion ist ein durch destillative Trennung gegenüber dem Einsatz-Gemisch hinsichtlich seiner Zusammensetzung verändertes Produkt zu verstehen, bei dem niedriger und/oder höher siedenden Komponenten abgetrennt wurden. Im konkreten Beispiel einer Trennwandkolonne nach Fig. 1 werden die Produktströme (A), (B) und (C) als Fraktionen bezeichnet. Werden beim erfindungsgemäßen Verfahren zwei oder drei der genannten Wertprodukte gewonnen, so erfolgt die Auftrennung in mehr als drei Fraktionen.

Für die weiteren Betrachtungen ist es zweckmäßig, das erfindungsgemäße Verfahren in sogenannte Destillationsblöcke aufzuteilen. Unter einem Destillationsblock ist eine Anordnung zu verstehen, welche mindestens eine Destillationskolonne enthält, wobei auch das Vorhandensein weiterer Apparate, wie beispielsweise Pumpen, Wärmetauscher oder Behälter möglich ist. Das erfindungsgemäße Verfahren umfaßt mindestens einen Destillationsblock aus der Reihe
(B1) Destillationsblock zur Abtrennung von Tetrahydrofuran
(B2) Destillationsblock zur Abtrennung von γ-Butyrolacton
(B3) Destillationsblock zur Abtrennung von 1,4-Butandiol,
wobei die Wertprodukte bevorzugt entsprechend ihrer Siedepunkte in der Reihenfolge (B1) vor (B2) vor (B3) gewonnen werden. Da die Siedepunkte der abzutrennenden Komponenten in dieser Reihenfolge steigen, können die Betriebsdrücke der Destillationskolonnen im allgemeinen fallend von (B1) über (B2) nach (B3) gewählt werden.

In Abhängigkeit von der Zusammensetzung des zu trennenden Gemisches und den gewünschten Anforderungen kann die destillative Trennung einen Destillationsblock (B1 oder B2 oder B3), zwei Destillationsblöcke (B1-B2 oder B1-B3 oder B2-B3) oder drei Destillationsblöcke (B1-B2-B3) enthalten. Es ist auch möglich, von dieser Reihenfolge abzuweichen. Die verschiedenen Destillationsblöcke sind zwingend durch den Massenstrom des zu trennenden Gemisches miteinander verbunden. Es ist möglich, Massenströme in vorherige oder nachfolgende Destillationsblöcke rück- oder weiterzuführen. Des weiteren ist auch eine energetische Verschaltung der verschiedenen Destillationsblöcke möglich und gegebenenfalls vorteilhaft.

### (B1) Destillationsblock zur Abtrennung von Tetrahydrofuran

Bevorzugt umfaßt der Destillationsblock zur Abtrennung von Tetrahydrofuran beim erfindungsgemäßen Verfahren mindestens zwei Destillationskolonnen, wobei der Druck in der Destillationskolonne, in der die Abtrennung des Tetrahydrofuran-Reinprodukts erfolgt, höher ist als in allen anderen Destillationskolonnen von (B1). Ähnlich den Ausführungen in der Offenlegungsschrift WO 91/01981 wird vorteilhafterweise zunächst bei einem niedrigen Druck eine mit Tetrahydrofuran sowie mit Wasser und/oder mit Veresterungsalkohol (sofern Maleinsäureester eingesetzt) angereicherte Fraktion gewonnen. Sie wird anschließend bei höherem Druck erneut destilliert, wobei Tetrahydrofuran hoher Reinheit gewonnen werden kann (Tetrahydrofuran-Reinprodukt). Die Leichtsiederfraktion der bei höherem Druck betriebenen Destillationskolonne, welche das nicht abgetrennte Tetrahydrofuran sowie das Wasser und/oder den Veresterungsalkohol enthält, wird vorteilhafterweise zur ersten Destillationskolonnne rückgeführt.

Beim erfindungsgemäßen Verfahren sind die folgenden drei Anordnungen der Destillationskolonnen bevorzugt.
(a) Trennwandkolonne (oder thermisch gekoppelte konventionelle Destillationskolonnen)/Trennwandkolonne (oder thermisch gekoppelte konventionelle Destillationskolonnen)
   In der ersten bevorzugten Anordnungen umfaßt der Destillationsblock (B1) zwei Trennwandkolonnen und/oder eine entsprechende Anordnung von thermisch gekoppelten konventionellen Destillationskolonnen, wobei
   (i) in der ersten Trennwandkolonne oder der entsprechenden Anordnung von thermisch gekoppelten konventionellen Destillationskolonnen (K1a) eine Trennung in mindestens drei Fraktionen erfolgt,
   (ii) die erhaltene, mit Tetrahydrofuran angereicherte Mittelsiederfraktion in der zweiten Trennwandkolonne oder der entsprechenden Anordnung von thermisch gekoppelten konventionellen Destillationskolonnen (K1b) in mindestens drei Fraktionen getrennt wird, wobei als Mittelsiederfraktion Tetrahydrofuran gewonnen wird und die Leichtsiederfraktion zu (K1a) rückgeführt wird.

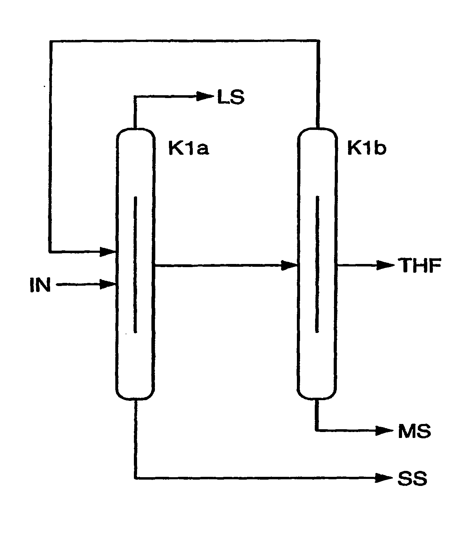

   Eine schematische Zeichnung der bevorzugten Verschaltung ist in Fig. 4 gegeben. Es sei darauf hingewiesen, daß auch das Vorhandensein weiterer Apparate, wie beispielsweise Pumpen, Wärmetauscher oder Behälter möglich ist. Die aus der Anordnung zu- oder abgeführten Massenströme sind wie folgt bezeichnet:
   (IN) Zulauf zum Destillationsblock (B1). In einer bevorzugten Ausführungsform entspricht der Zulauf dem bei der Hydrierung von Maleinsäureanhydrid, Maleinsäure, deren Ester oder Halbester anfallendem Gemisch.
   (THF) Tetrahydrofuran hoher Reinheit.
   (LS) Leichtsieder, enthaltend Nebenprodukte.
   (MS) Mittelsieder, enthaltend Nebenprodukte.
   (SS) Schwersieder, enthaltend Nebenprodukte und im allgemeinen γ-Butyrolacton und 1,4-Butandiol. Zur Gewinnung der weiteren Wertprodukte wird diese Fraktion im allgemeinen dem Destillationsblock (B2) oder (B3) zugeführt.

   Aus dem Zulauf (IN) wird in (K1a) eine mit Tetrahydrofuran angereicherte Mittelsiederfraktion gewonnen. Sie enthält überwiegend Tetrahydrofuran sowie Wasser und/oder den bei der Hydrierung als Esterkomponente eingesetzten Alkohol. Sie wird anschließend bei höherem Druck in (K1b) zur Gewinnung des Tetrahydrofurans hoher Reinheit (THF) rektifiziert. Über den Kopf von (K1b) wird ein Gemisch, welches nicht-abgetrenntes Tetrahydrofuran sowie Wasser und/oder den genannten Alkohol enthält, abgezogen und erneut (K1a) zugeführt. Die Einspeisung dieses Rückstromes in (K1a) kann zusammen mit dem Zulauf (IN) erfolgen. Hinsichtlich des Energiebedarfs ist es jedoch vorteilhafter, eine separate Einspeisung vorzusehen, welche bevorzugt oberhalb des Zulaufs (IN) liegt. Die Fraktionen (LS) und (MS) werden im allgemeinen aus dem Destillationsblock ausgeschleust. Gegebenenfalls ist es vorteilhaft, einen Teil von (MS) zu (K1a) rückzuführen. Die Schwersiederfraktion (SS) wird im allgemeinen dem nachfolgenden Destillationsblock zur weiteren Aufarbeitung zugeführt.
(b) konventionelle Destillationskolonne /Trennwandkolonne (oder thermisch gekoppelte konventionelle Destillationskolonnen)
   In der zweiten bevorzugten Anordnungen umfaßt der Destillationsblock (B1) eine vorgeschaltete konventionelle Destillationskolonne und eine Trennwandkolonne oder eine entsprechende Anordnung von thermisch gekoppelten konventionellen Destillationskolonnen, wobei
   (i) in der ersten, konventionellen Destillationskolonne eine Trennung in mindestens zwei Fraktionen erfolgt,
   (ii) die erhaltene, mit Tetrahydrofuran angereicherte Kopffraktion in der Trennwandkolonne oder der entsprechenden Anordnung von thermisch gekoppelten konventionellen Destillationskolonnen (K1b) in mindestens drei Fraktionen getrennt wird, wobei als Mittelsiederfraktion Tetrahydrofuran gewonnen wird und die Leichtsiederfraktion zur konventionellen Destillationskolonne rückgeführt wird.

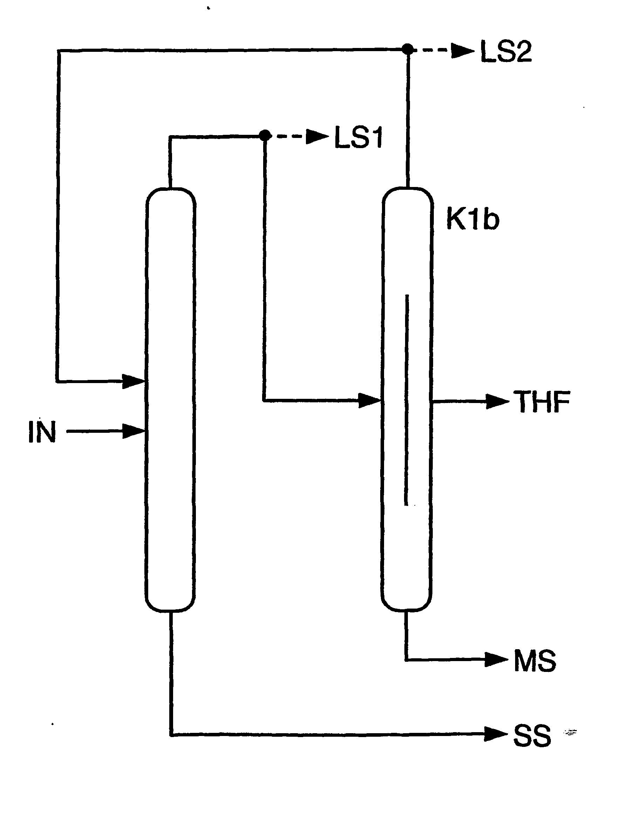

   Eine schematische Zeichnung der bevorzugten Verschaltung ist in Fig. 5 gegeben. Es sei darauf hingewiesen, daß auch das Vorhandensein weiterer Apparate, wie beispielsweise Pumpen, Wärmetauscher oder Behälter möglich ist. Die aus der Anordnung zu- oder abgeführten Massenströme sind wie folgt bezeichnet:
   (IN) Zulauf zum Destillationsblock (B1). In einer bevorzugten Ausführungsform entspricht der Zulauf dem bei der Hydrierung von Maleinsäureanhydrid, Maleinsäure, deren Ester oder Halbester anfallendem Gemisch.
   (THF) Tetrahydrofuran hoher Reinheit.
   (LS1) Leichtsieder, enthaltend Nebenprodukte.
   (LS2) Leichtsieder, enthaltend Nebenprodukte.
   (MS) Mittelsieder, enthaltend Nebenprodukte.
   (SS) Schwersieder, enthaltend Nebenprodukte und im allgemeinen γ-Butyrolacton und 1,4-Butandiol. Zur Gewinnung der weiteren Wertprodukte wird diese Fraktion im allgemeinen dem Destillationsblock (B2) oder (B3) zugeführt.

   Bei dieser Anordnung kommen zwei Stellen zum Abzug der Leichtsieder in Betracht. Je nach Ausführungsform ist es möglich, über (LS1), über (LS2) oder parallel über beide Stellen die Leichtsieder zu entfernen. Vorzugsweise erfolgt die Entfernung der Leichtsieder über (LS1) oder (LS2).
   Aus dem Zulauf (IN) wird in der ersten Destillationskolonne eine mit Tetrahydrofuran angereicherte Leichtsiederfraktion gewonnen. Sie enthält überwiegend Tetrahydrofuran sowie Wasser und/oder den bei der Hydrierung als Esterkomponente eingesetzten Alkohol. Sie wird anschließend bei höherem Druck in (K1b) zur Gewinnung des Tetrahydrofurans hoher Reinheit (THF) rektifiziert. Über Kopf von (K1b) wird ein Gemisch, welches nicht-abgetrenntes Tetrahydrofuran sowie Wasser und/oder den genannten Alkohol enthält, abgezogen und erneut der ersten Destillationskolonne zugeführt. Die Einspeisung dieses Rückstromes in die erste Destillationskolonne kann zusammen mit dem Zulauf (IN) erfolgen. Hinsichtlich des Energiebedarfs ist es jedoch vorteilhafter, eine separate Einspeisung vorzusehen, welche bevorzugt oberhalb des Zulaufs (IN) liegt. Die Fraktionen (LS1), (LS2) und (MS) werden im allgemeinen aus dem Destillationsblock ausgeschleust. Gegebenenfalls ist es vorteilhaft, einen Teil von (MS) zur ersten Destillationskolonne rückzuführen. Die Schwersiederfraktion (SS) wird im allgemeinen dem nachfolgenden Destillationsblock zur weiteren Aufarbeitung zugeführt.
(c) Trennwandkolonne (oder thermisch gekoppelte konventionelle Destillationskolonnen)/zwei hintereinandergeschaltete konventionelle Destillationskolonnen
   In der dritten bevorzugten Anordnungen umfaßt der Destillationsblock (B1) eine vorgeschaltete Trennwandkolonne oder eine entsprechende Anordnung von thermisch gekoppelten konventionellen Destillationskolonnen und nachfolgend zwei hintereinandergeschaltete konventionelle Destillationskolonnen, wobei
   (i) in der vorgeschalteten Trennwandkolonne oder der entsprechenden Anordnung von thermisch gekoppelten konventionellen Destillationskolonnen (K1a) eine Trennung in mindestens drei Fraktionen erfolgt,
   (ii) die erhaltene, mit Tetrahydrofuran angereicherte Mittelsiederfraktion in der nachfolgenden konventionellen Destillationskolonne in mindestens zwei Fraktionen getrennt wird, wobei die Leichtsiederfraktion zu (K1a) rückgeführt wird und die mit Tetrahydrofuran angereicherte Schwersiederfraktion
   (iii) in der zweiten, konventionellen Destillationskolonne in mindestens zwei Fraktionen getrennt wird, wobei als Leichtsiederfraktion Tetrahydrofuran gewonnen wird.

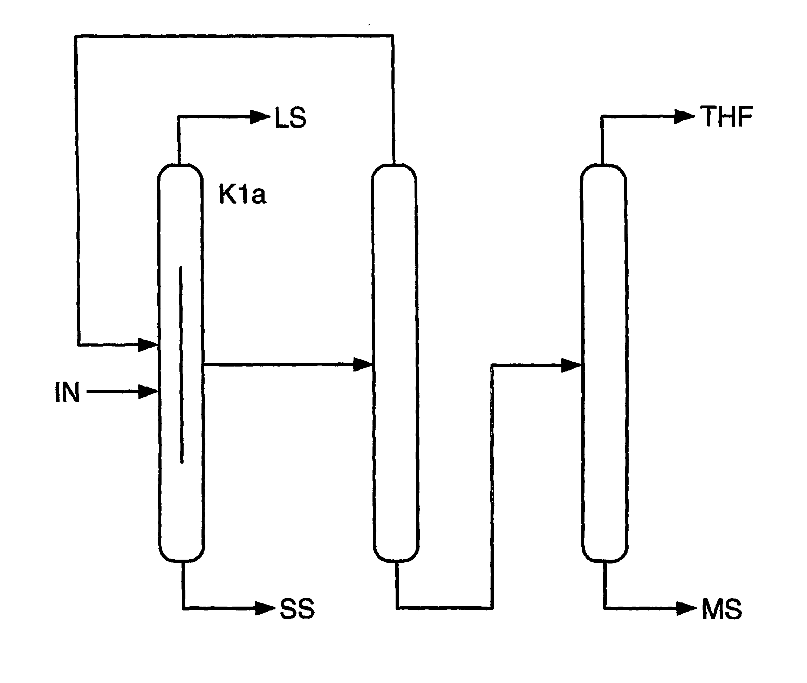

   Eine schematische Zeichnung der bevorzugten Verschaltung ist in Fig. 6 gegeben. Es sei darauf hingewiesen, daß auch das Vorhandensein weiterer Apparate, wie beispielsweise Pumpen, Wärmetauscher oder Behälter möglich ist. Die aus der Anordnung zu- oder abgeführten Massenströme sind wie folgt bezeichnet:
   (IN) Zulauf zum Destillationsblock (B1). In einer bevorzugten Ausführungsform entspricht der Zulauf dem bei der Hydrierung von Maleinsäureanhydrid, Maleinsäure, deren Ester oder Halbester anfallendem Gemisch.
   (THF) Tetrahydrofuran hoher Reinheit.
   (LS) Leichtsieder, enthaltend Nebenprodukte.
   (MS) Mittelsieder, enthaltend Nebenprodukte.
   (SS) Schwersieder, enthaltend Nebenprodukte und im allgemeinen γ-Butyrolacton und 1,4-Butandiol. Zur Gewinnung der weiteren Wertprodukte wird diese Fraktion im allgemeinen dem Destillationsblock (B2) oder (B3) zugeführt.

   Aus dem Zulauf (IN) wird in (K1a) eine mit Tetrahydrofuran angereicherte Mittelsiederfraktion gewonnen. Sie enthält überwiegend Tetrahydrofuran sowie Wasser und/oder den bei der Hydrierung als Esterkomponente eingesetzten Alkohol. Sie wird der nachfolgenden konventionellen Destillationskolonne zugeführt, welche vorteilhafterweise bei einem höheren Druck betrieben wird. Über Kopf wird ein Gemisch, welches nicht-abgetrenntes Tetrahydrofuran sowie Wasser und/oder den genannten Alkohol enthält, abgezogen und erneut (K1a) zugeführt. Die Einspeisung dieses Rückstromes in die erste Destillationskolonne kann zusammen mit dem Zulauf (IN) erfolgen. Hinsichtlich des Energiebedarfs ist es jedoch vorteilhafter, eine separate Einspeisung vorzusehen, welche bevorzugt oberhalb des Zulaufs (IN) liegt. Die Schwersiederfraktion der ersten konventionellen Destillationskolonne wird der zweiten konventionellen Destillationskolonne zugeführt, wobei in dieser die Gewinnung des Tetrahydrofurans hoher Reinheit (THF) über Kopf erfolgt. Die Fraktionen (LS) und (MS) werden im allgemeinen aus dem Destillationsblock ausgeschleust. Gegebenenfalls ist es vorteilhaft, einen Teil von (MS) zur ersten Destillationskolonne rückzuführen. Die Schwersiederfraktion (SS) wird im allgemeinen dem nachfolgenden Destillationsblock zur weiteren Aufarbeitung zugeführt.

Beim erfindungsgemäßen Verfahren besonders bevorzugt sind die beiden Verschaltungen (a) Trennwandkolonne (oder thermisch gekoppelte konventionelle Destillationskolonnen) / Trennwandkolonne (oder thermisch gekoppelte konventionelle Destillationskolonnen) und (b) konventionelle Destillationskolonne / Trennwandkolonne (oder thermisch gekoppelte konventionelle Destillationskolonnen).

Wird als erste Destillationskolonne eine Trennwandkolonne oder eine entsprechende Anordnung von thermisch gekoppelten konventionellen Destillationskolonnen (K1a) eingesetzt, wie unter (a) oder (c) beschrieben, so betreibt man diese bevorzugt bei einem Absolutdruck von 0,05 bis 0,2 MPa abs, besonders bevorzugt bei Atmosphärendruck.

Die einzelnen Teilbereiche in (K1a) sind im allgemeinen durch folgende theoretische Trennstufenzahlen charakterisiert:

| | |
|---|---|
| Kopf teil (1) : | bevorzugt 0 bis 60, |
| | besonders bevorzugt 10 bis 60; |
| oberer Zulaufteil (2): | bevorzugt 0 bis 60, |
| | besonders bevorzugt 10 bis 60; |
| oberer Entnahmeteil (3): | bevorzugt 0 bis 60, |
| | besonders bevorzugt 10 bis 60; |
| unterer Zulauf teil (4) : | bevorzugt 0 bis 30, |
| | besonders bevorzugt 1 bis 25; |
| unterer Entnahmeteil (5): | bevorzugt 0 bis 60, |
| | besonders bevorzugt 10 bis 50; |
| Sumpfteil (6): | bevorzugt 0 bis 60, |
| | besonders bevorzugt 1 bis 40. |

Die Summe der theoretischen Trennstufen in (K1a) des oberen und unteren Zulauf teils (2) und (4) betragen bevorzugt 80 bis 110 %, besonders bevorzugt 90 bis 100 % der Summe der theoretischen Trennstufen des oberen und unteren Entnahmeteils (3) und (5).

(K1b), welche als Trennwandkolonne oder als entsprechende Anordnung von thermisch gekoppelten konventionellen Destillationskolonnen ausgebildet ist und deren Einsatz beispielsweise in (a) und (b) beschrieben ist, betreibt man bevorzugt bei einem Absolutdruck von 0,3 bis 1,2 MPa abs, besonders bevorzugt bei 0,5 bis 0,7 MPa abs.

Die einzelnen Teilbereiche in (K1b) sind im allgemeinen durch folgende theoretische Trennstufenzahlen charakterisiert:

| | |
|---|---|
| Kopfteil (1) : | bevorzugt 0 bis 60, |
| | besonders bevorzugt 10 bis 60; |
| oberer Zulauf teil (2): | bevorzugt 0 bis 60, |
| | besonders bevorzugt 10 bis 60; |
| oberer Entnahmeteil (3): | bevorzugt 0 bis 60, |
| | besonders bevorzugt 10 bis 60; |
| unterer Zulaufteil (4): | bevorzugt 0 bis 60, |
| | besonders bevorzugt 10 bis 60; |
| unterer Entnahmeteil (5): | bevorzugt 0 bis 60, |
| | besonders bevorzugt 10 bis 60; |
| Sumpfteil (6): | bevorzugt 0 bis 60, |
| | besonders bevorzugt 10 bis 60. |

Die Summe der theoretischen Trennstufen in (K1b) des oberen und unteren Zulauf teils (2) und (4) betragen bevorzugt 80 bis 110 %, besonders bevorzugt 90 bis 100 % der Summe der theoretischen Trennstufen des oberen und unteren Entnahmeteils (3) und (5).

Wird als erste Destillationskolonne eine konventionelle Destillationskolonne eingesetzt, wie unter (b) beschrieben, so betreibt man diese bevorzugt bei einem Absolutdruck von 0,1 bis 0,15 MPa abs, besonders bevorzugt bei 0,10 bis 0,11 MPa abs. Der Teilbereich oberhalb des Zulaufs weist im allgemeinen 10 bis 60 theoretische Trennstufen, bevorzugt 20 bis 50 theoretische Trennstufen auf. Der Teilbereich unterhalb des Zulaufs weist im allgemeinen 5 bis 30 theoretische Trennstufen, bevorzugt 10 bis 25 theoretische Trennstufen auf.

Bei der unter (c) beschriebenen Anordnung mit einer vorgeschalteten Trennwandkolonne oder eine entsprechenden Anordnung von thermisch gekoppelten konventionellen Destillationskolonnen und nachfolgend zwei hintereinandergeschalteten konventionellen Destillationskolonnen, betreibt man die erste der beiden konventionellen Destillationskolonnen bevorzugt bei einem Absolutdruck von 0,5 bis 1,0 MPa abs, besonders bevorzugt bei 0,4 bis 0,8 MPa abs. Der Teilbereich oberhalb des Zulaufs weist im allgemeinen 0 bis 30 theoretische Trennstufen, bevorzugt 0 bis 20 theoretische Trennstufen auf. Der Teilbereich unterhalb des Zulaufs weist im allgemeinen 20 bis 50 theoretische Trennstufen, bevorzugt 20 bis 40 theoretische Trennstufen auf. Die zweite der beiden konventionellen Destillationskolonnen betreibt man bevorzugt bei einem niedrigeren Druck als die erste. Bevorzugt ist ein Absolutdruck von 0,1 bis 0,15 MPa abs, besonders bevorzugt von 0,10 bis 0,11 MPa abs und ganz besonders bevorzugt Atmosphärendruck. Der Teilbereich oberhalb des Zulaufs weist im allgemeinen 10 bis 30 theoretische Trennstufen, bevorzugt 15 bis 25 theoretische Trennstufen auf. Der Teilbereich unterhalb des Zulaufs weist im allgemeinen 20 bis 40 theoretische Trennstufen, bevorzugt 25 bis 35 theoretische Trennstufen auf.

### (B2) Destillationsblock zur Abtrennung von γ-Butyrolacton

Das γ-Butyrolacton enthaltende Gemisch, welches dem Destillationsblock (B2) zugeführt wird, entstammt beispielsweise aus dem Destillationsblock (B1). Es ist jedoch auch möglich, daß das Gemisch aus anderen Aufarbeitungseinheiten, z.B. Destillationsblock (B3), oder direkt aus dem Herstellverfahren des Gemisches, z.B. der Hydrierung von Maleinsäureanhydrid, Maleinsäure, deren Ester oder Halbester, stammt.

Bevorzugt umfaßt der Destillationsblock zur Abtrennung von γ-Butyrolacton beim erfindungsgemäßen Verfahren eine Trennwandkolonne oder eine entsprechende Anordnung von thermisch gekoppelten konventionellen Destillationskolonnen (K2). In diesem Destillationsblock wird γ-Butyrolacton in einer Seitenentnahmestelle (B) bevorzugt in flüssiger Form entnommen. Am Kopf der Kolonne werden leichtsiedende Nebenprodukte (A) entnommen. Je nach Zusammensetzung des Zulaufgemisches und Ausführungsform der destillativen Trennung werden diese leichtsiedenden Produkte ganz oder teilweise ausgeschleust oder in die der destillativen Aufarbeitung vorausgehenden Synthesestufen zurückgeführt.

### (K2) wird bevorzugt bei einem Absolutdruck von 0,001 bis 0,15 MPa abs, besonders bevorzugt bei 0,01 bis 0,02 MPa abs betrieben.

Die einzelnen Teilbereiche in (K2) sind im allgemeinen durch folgende theoretische Trennstufenzahlen charakterisiert:

| | |
|---|---|
| Kopfteil (1) : | bevorzugt 10 bis 100, |
| | besonders bevorzugt 15 bis 95; |
| oberer Zulauf teil (2): | bevorzugt 10 bis 100, |
| | besonders bevorzugt 15 bis 95; |
| oberer Entnahmeteil (3): | bevorzugt 10 bis 100, |
| | besonders bevorzugt 15 bis 95; |
| unterer Zulauf teil (4): | bevorzugt 10 bis 100, |
| | besonders bevorzugt 15 bis 95; |
| unterer Entnahmeteil (5): | bevorzugt 10 bis 100, |
| | besonders bevorzugt 15 bis 95; |
| Sumpfteil (6): | bevorzugt 10 bis 100, |
| | besonders bevorzugt 15 bis 95. |

Die Summe der theoretischen Trennstufen in (K2) des oberen und unteren Zulauf teils (2) und (4) betragen bevorzugt 80 bis 110 %, besonders bevorzugt 90 bis 100 % der Summe der theoretische Trennstufen des oberen und unteren Entnahmeteils (3) und (5).

### (B3) Destillationsblock zur Abtrennung von 1,4-Butandiol

Das 1,4-Butandiol enthaltende Gemisch, welches dem Destillationsblock (B3) zugeführt wird, entstammt beispielsweise aus dem Destillationsblock (B2). Es ist jedoch auch möglich, daß das Gemisch aus anderen Aufarbeitungseinheiten, z.B. Destillationsblock (B1), oder direkt aus dem Herstellverfahren des Gemisches, z.B. der Hydrierung von Maleinsäureanhydrid, Maleinsäure, deren Ester oder Halbester, stammt.

Bevorzugt umfaßt der Destillationsblock zur Abtrennung von 1,4-Butandiol beim erfindungsgemäßen Verfahren eine Trennwandkolonne oder eine entsprechende Anordnung von thermisch gekoppelten konventionellen Destillationskolonnen (K3). In diesem Destillationsblock wird 1,4-Butandiol in einer Seitenentnahmestelle (B) bevorzugt in flüssiger Form entnommen. Am Kopf der Kolonne werden leichtsiedende Nebenprodukte (A) entnommen. Je nach Zusammensetzung des Zulaufgemisches und Ausführungsform der destillativen Trennung werden diese leichtsiedenden Produkt ganz oder teilweise ausgeschleust oder in die vorangehenden Destillationsblöcke (B1) oder (B2) zurückgeführt. Es ist auch möglich, in einer optionalen, nachgeschalteten Destillationskolonne oder einem Verdampfer, bevorzugt einem Fallfilmverdampfer oder einem Dünnschichtverdampfer, das Sumpfprodukt (C) weiter einzuengen und auf diese Weise weitere Mengen an 1,4-Butandiol zurückzugewinnen.

### (K3) wird bevorzugt bei einem Absolutdruck von 0,001 bis 0,05 MPa abs, besonders bevorzugt bei 0,003 bis 0,01 MPa abs betrieben.

Die einzelnen Teilbereiche in (K3) sind im allgemeinen durch folgende theoretische Trennstufenzahlen charakterisiert:

| | |
|---|---|
| Kopf teil (1): | bevorzugt 10 bis 100, |
| | besonders bevorzugt 15 bis 95; |
| oberer Zulauf teil (2): | bevorzugt 10 bis 100, |
| | besonders bevorzugt 15 bis 95; |
| oberer Entnahmeteil (3): | bevorzugt 10 bis 100, |
| | besonders bevorzugt 15 bis 95; |
| unterer Zulauf teil (4): | bevorzugt 10 bis 100, |
| | besonders bevorzugt 15 bis 95; |
| unterer Entnahmeteil (5): | bevorzugt 10 bis 100, |
| | besonders bevorzugt 15 bis 95; |
| Sumpfteil (6): | bevorzugt 10 bis 100, |
| | besonders bevorzugt 15 bis 95. |

Die Summe der theoretischen Trennstufen in (K3) des oberen und unteren Zulaufteils (2) und (4) betragen bevorzugt 80 bis 110 %, besonders bevorzugt 90 bis 100 % der Summe der theoretischen Trennstufen des oberen und unteren Entnahmeteils (3) und (5).

Alle beim erfindungsgemäßen Verfahren einzusetzenden Trennwandkolonnen und thermisch gekoppelten konventionellen Destillationskolonnen können sowohl mit Destillationspackungen als auch mit Destillationsböden als Kolonneneinbauten ausgerüstet werden. Explizit genannt seien geordnete Packungen, Füllkörper, Ventilböden, Siebböden oder Glockenböden.

Werden hohe Anforderungen an die Reinheit der Produkte gestellt, so ist es vorteilhaft, Destillationspackungen einzusetzen. Aufgrund der spezifischen Trenneigenschaften ist es besonders vorteilhaft, geordnete Gewebe- oder Blechpackungen zu verwenden.

Bei Destillationskolonnen mit einem inneren Durchmesser von mehr als 1,2 m ist es aus wirtschaftlichen Erwägungen (Investitionskosten) empfehlenswert, Destillationsböden einzusetzen, sofern keine übergeordneten Gründe entgegenstehen. Bei konventionellen Destillationskolonnen sind Ventilböden, Siebböden und Glockenböden geeignet, bei Trennwandkolonnen Ventilböden und Siebböden.

Beim erfindungsgemäßen Verfahren werden in (K1a), (K1b), (K2) und (K3) bevorzugt geordnete Packungen, Füllkörper, Ventilböden, Siebböden und/oder Glockenböden verwendet, deren Druckverlust stetig mit zunehmender Gasbelastung um mindestens 20 % pro Erhöhung des F-Faktors um 0,5 Pa^{0,5} zunimmt. Unter dem F-Faktor ist das Produkt aus der Gasgeschwindigkeit und der Wurzel der Gasdichte zu verstehen (siehe Ullmann's Encyclopedia of Industrial Chemistry, 6th edition, 1999 Electronic Release, Chapter "Distillation and Rectification, Design and Dimensioning"). Durch das bevorzugte Druckverlustverhalten wird eine vorteilhafte Aufteilung des aufsteigenden Gasstroms aus dem Sumpfteil (6) in den unteren Zulaufteil (4) und den unteren Entnahmeteil (5) erreicht.

Aufgrund der hervorragenden Trenneigenschaften werden beim erfindungsgemäßen Verfahren in (K2) und (K3) besonders bevorzugt geordnete Packungen und/oder Füllkörper eingesetzt, deren Druckverlust stetig mit zunehmender Gasbelastung um mindestens 20 % pro Erhöhung des F-Faktors um 0,5 Pa^{0,5} zunimmt. Durch das bevorzugt Druckverlustverhalten wird eine vorteilhafte Aufteilung des aufsteigenden Gasstroms aus dem Supfteil (6) in den unteren Zulaufteil (4) und den unteren Entnahmeteil (5) erreicht.

Bei besonders hohen Anforderungen an die Reinheit der Produkte ist es vorteilhaft, die Trennwand mit einer thermischen Isolierung zu versehen. Eine Beschreibung verschiedener Möglichkeiten findet sich beispielsweise in der Patentschrift US 5,785,819. Besonders vorteilhaft ist eine Ausführung mit einem zwischenliegenden engen Gasraum. Beim erfindungsgemäßen Verfahren wird daher die Trennwand in (K2) und (K3) besonders bevorzugt wärmeisoliert ausgeführt.

Wie oben beschrieben und in Fig. 1 bis 3 dargestellt, kann der Bereich innerhalb der Trennwandkolonne oder der entsprechenden Anordnung von thermisch gekoppelten konventionellen Destillationskolonnen grob in die sechs Teilbereiche (1) bis (6) unterteilt werden. In Abhängigkeit von der lokal in der Destillationskolonne vorliegenden Trennaufgabe ist es möglich, verschiedene Einbauten innerhalb einer Kolonne einzusetzen. So kann eine Trennwandkolonne oder eine entsprechende Anordnung von thermisch gekoppelten konventionellen Destillationskolonnen verschiedene Destillationspackungen, verschiedenen Destillationsböden oder auch beides enthalten. Auch innerhalb eines der sechs Teilbereiche sind verschiedene Destillationspackungen möglich.

Gegenstand der Erfindung ist weiterhin ein Regelkonzept zur Regelung des erfindungsgemäßen Verfahrens.

Für die Regelung von Trennwandkolonnen und entsprechender Anordnungen von thermisch gekoppelten konventionellen Destillationskolonnen sind verschiedene Regelungsstrategien vorbeschrieben.

Bei sehr einfachen Trennaufgaben, beispielsweise bei sich ideal verhaltenden Zweistoffgemischen in einer konventionellen Destillationskolonne, ist in vielen Fällen eine mathematische Modellierung möglich. Sind noch weitere Komponenten zugegen oder verhält sich das System nicht ideal, so wird bereits in vielen Fällen kein befriedigendes Simulationsmodell mehr erhalten. Noch komplexer ist die mathematische Modellierung der Trennung eines Mehrstoffsystems in einer Trennwandkolonne oder einer entsprechenden Anordnung von thermisch gekoppelten konventionellen Destillationskolonnen. Bisher bekanntgewordene Modellrechnungen verdeutlichen eher die möglicherweise auftretenden Instabilitätsprobleme beim Betrieb als daß sie praktisch verwertbare Hinweise zum optimalen Regeln einer Destillationskolonne geben. Daher werden vielfach unter Zugrundelegung von Plausibilitätsbetrachtungen und experimenteller Ergebnisse Vereinfachungen der Modelle vorgenommen.

So beschreibt M. Knott, Process Engineering, Vol. 2, 1993, February, Seite 33 bis 34 ein vereinfachtes Simulationsmodell, bei dem die Zusammensetzung des Kopfprodukts über die Entnahmemenge des Seitenprodukts, die Zusammensetzung des Seitenprodukts über das Rücklaufverhältnis und die Zusammensetzung des Sumpfprodukts über dessen Entnahmemenge geregelt wird. Zur Durchführung der Regelung ist dennoch ein komplexes, dynamisches Computermodell erforderlich. Erfindungsgemäß wurde erkannt, daß bei der vorliegenden Aufgabenstellung, nämlich der destillativen Trennung von Gemischen, die bei der Hydrierung von Maleinsäureanhydrid, Maleinsäure, deren Ester oder Halbester anfallen, in mindestens drei Fraktionen, wobei mindestens eine Fraktion Tetrahydrofuran, γ-Butyrolacton und/oder 1,4-Butandiol enthält, durch das vorbeschriebene Regelungskonzept weder die erforderliche hohe Reinheit der Wertprodukte in den Seitenabzügen noch die erforderliche Stabilität bei Schwankungen in der Menge der Produktströme und deren Zusammensetzung gewährleistet werden kann.

In E.A. Wolff et al., Ind. Eng. Chem. Res. 34, 1995, Seiten 2094 bis 2103 wird über die Modellierung zur Trennung von Dreistoffgemischen in Trennwandkolonnen berichtet. Darin wird erkannt, daß vor allem in den Fällen, in denen eine hohe Produktreinheit angestrebt wird, gravierende Probleme zu erwarten sind. Als Lösungsvorschlag wird eine umfassende mathematische Analyse angegeben, die für jeden Einzelfall durchzuführen ist. Es versteht sich wohl von selbst, da ein derartiges Regelungskonzept zur Trennung eines Vielstoffgemisches in der Praxis nicht einsetzbar ist.

Das US-Patent 4,230,533 lehrt die Regelung einer Trennwandkolonne über die Konzentration der Leichtsiederkomponente im unteren Zulaufteil als Stellgröße für die Aufteilung des relativen Gas- und Flüssigkeitsstromes zwischen unteren Zulaufteil und unteren Entnahmeteil. Erfindungsgemäß wurde erkannt, daß dieses vereinfachte Regelungskonzept weder die bei der vorliegenden Aufgabenstellung erforderliche hohe Reinheit der Wertprodukte in den Seitenabzügen noch die erforderliche Stabilität bei Schwankungen in der Menge der Produktströme und deren Zusammensetzung gewährleisten kann.

In der Offenlegungsschrift DE-A 35 22 234 ist ein Verfahren zum energiegünstigen Betreiben einer Trennwandkolonne beschrieben. Kern der Offenbarung ist eine gezielte Aufteilung des Flüssigkeitsstroms am oberen Ende der Trennwand zwischen oberen Zulaufteil und oberen Entnahmeteil. Aus dem Zulauf teil dürfen nach oben nur Leichtsieder und Mittelsieder und nach unten nur Schwersieder und Mittelsieder austreten. Nur unter diesen Bedingungen ist ein energiegünstiger Betrieb bei gleichzeitiger Erfüllung hoher Reinheitsanforderungen möglich. Die Aufteilung des Flüssigkeitsstroms wird über die vier Temperaturen des oberen und unteren Zulaufteils und des oberen und unteren Entnahmeteils als Meßgröße geregelt. Erfindungsgemäß wurde erkannt, daß auch dieses Regelungskonzept weder die bei der vorliegenden Aufgabenstellung erforderliche hohe Reinheit der Wertprodukte in den Seitenabzügen noch die erforderliche Stabilität bei Schwankungen in der Menge der Produktströme und deren Zusammensetzung gewährleisten kann.

Die Offenlegungsschrift EP-A 0 780 147 offenbart ein Konzept zur Regelung einer Trennwandkolonne oder einer entsprechenden Anordnung von thermisch gekoppelten konventionellen Destillationskolonnen, welches auf die Messung von Konzentrationen der zu trennenden Komponenten im Bereich der Destillationskolonne zurückgreift und aus diesen Werten sogenannte Regelungseingriffe bildet.

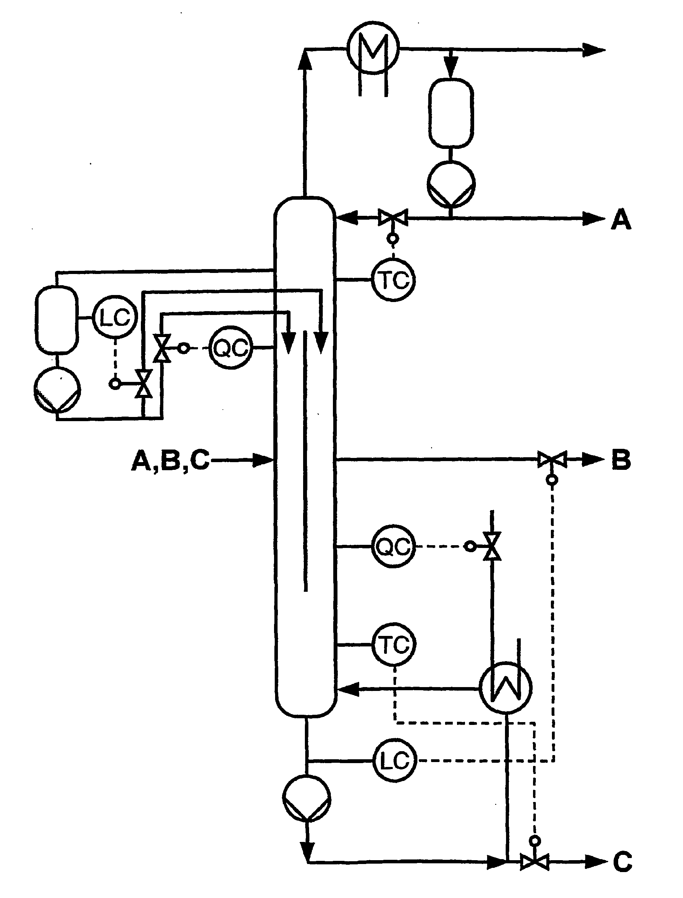

Das erfindungsgemäße Regelungskonzept der beim erfindungsgemäßen Verfahren eingesetzten Trennwandkolonnen und/oder der entsprechenden Anordnung von thermisch gekoppelten konventionellen Destillationskolonnen kann bei allen, im erfindungsgemäßen Verfahren eingesetzten Trennwandkolonnen und/oder der entsprechenden Anordnungen von thermisch gekoppelten konventionellen Destillationskolonnen verwendet werden. Bevorzugt wird das erfindungsgemäße Regelungskonzept eingesetzt für die Kolonnen (K1a), (K1b) (K2) und (K3).
(a) Das Verhältnis des ablaufenden Flüssigkeitsstromes am oberen Ende des oberen Zulauf teils (2) zum ablaufenden Flüssigkeitsstrom am oberen Ende des oberen Entnahmeteils (3) beträgt bevorzugt 0,1 bis 1,0, besonders bevorzugt 0,3 bis 0,6. Daraus ermöglicht sich eine mittige Anordnung der Trennwand, was zu konstruktiven Vorteilen führt.
   Die gezielte Aufteilung des am Kopfteil (1) ablaufenden Flüssigkeitsstroms wird bevorzugt dadurch realisiert, daß man den Flüssigkeitsstrom in einem innerhalb oder außerhalb der Destillationskolonne angeordneten Auffangraum sammelt und durch eine Festeinstellung oder eine Regelung aufteilt. Darin enthalten ist auch die Funktion zur Weiterleitung der Flüssigkeit, beispielsweise durch die Funktion einer Pumpenvorlage oder der Sicherstellung einer ausreichend hohen statischen Flüssigkeitshöhe. Die gezielte Aufteilung kann beispielsweise über mengengeregelte Pumpen und/oder mengengeregelte Stellorgane, beispielsweise Ventile, erfolgen. Die aufgeteilten Flüssigkeitsströme werden nun in die beiden Kolonnenbereiche (2) und (3) geleitet. Bei Destillationskolonnen mit Destillationsböden als Einbauten (sogenannte Bodenkolonnen) ist es besonders günstig, hierzu den Ablaufschacht auf etwa das Zwei- bis Dreifache der üblichen Höhe zu vergrößern und in ihm die entsprechende Flüssigkeitsmenge zu speichern. Bei Destillationskolonnen mit Destillationspackungen als Einbauten (sogenannte Packungskolonnen) wird die Flüssigkeit zunächst in Sammlern gefaßt und von dort aus in einen innenliegenden oder außenliegenden Auffangraum geleitet.
   Eine nicht limitierende, schematische Darstellung zur Einbindung und Regelung ist in Fig. 7 gegeben.
(b) Das Verhältnis des Brüdenstrom am unteren Ende des unteren Zulauf teils (4) zum Brüdenstrom am unteren Ende des unteren Entnahmeteils (5) beträgt bevorzugt 0,8 bis 1,2, besonders bevorzugt 0,85 bis 1,15. Daraus ermöglicht sich eine vorteilhafte mittige Anordnung der Trennwand.
   Die gezielte Aufteilung des am Sumpfteil (6) emporsteigenden Brüdenstroms wird bevorzugt durch die Wahl geeigneter Trenneinbauten und/oder den zusätzlichen Einbau druckverlusterzeugender Einbauten, wie beispielsweise Blenden, realisiert.
(c) Der Flüssigkeitsstrom auf die Zulaufstelle wird über eine Mengenregelung bevorzugt derart eingestellt, daß der auf den Zulaufteil (2) und (4) insgesamt aufgegebene Flüssigkeitsstrom nicht unter 30 % des Auslegungswertes sinken kann. Unter dem Auslegungswert ist der Wert zu verstehen, für den die Kolonne thermodynamisch und fluiddynamisch ausgelegt beziehungsweise berechnet wurde.
   Durch diese Maßnahme wird die Destillationskolonne in einem noch stabilen Arbeitsbereich gehalten und die hohe Reinheit der Wertprodukte sichergestellt.
   Die erfindungsgemäße Maßnahme der Mengenregelung wird bevorzugt dadurch sichergestellt, daß man den Flüssigkeitsstrom auf die Zulaufstelle über eine Pumpvorrichtung oder über eine statische Zulaufhöhe von mindestens 1 m aus einer Zwischenvorlage mengengeregelt aufgibt. Dadurch werden vor allem kurzzeitige Störungen in der Aufgabenmenge vermieden, welche sonst zu teilweise empfindlichen Störungen des Betriebszustandes der Destillationskolonne führen könnten und somit die kontinuierlich hohe Reinheit der Wertprodukte gefährden könnten.
(d) Der ablaufende Flüssigkeitsstrom am unteren Ende des oberen Entnahmeteils (3) wird über eine Regelung bevorzugt derart zwischen dem abgeführten Flüssigkeitsstrom der Entnahmestelle und dem, auf den unteren Entnahmeteil (5) aufgegebenen Flüssigkeitsstrom aufgeteilt, daß der auf dem unteren Entnahmeteil (5) aufgegebene Flüssigkeitsstrom nicht unter 30 % des Auslegungswertes sinken kann. Unter dem Auslegungswert ist der Wert zu verstehen, für den die Kolonne thermodynamisch und fluiddynamisch ausgelegt beziehungsweise berechnet wurde.
   Bei der Trennung von Mehrstoffgemischen in eine Leichtsieder-, Mittelsieder- und Schwersiederfraktion existieren üblicherweise Spezifikationen über den maximal zulässigen Anteil an Leichtsiedern und Schwersiedern in der Mittelsiederfraktion. Hierbei werden entweder einzelne für das Trennproblem kritische Komponenten, sogenannte Schlüsselkomponenten, oder die Summe von mehreren Schlüsselkomponenten spezifiziert. In den Punkten (e) und (f) ist die erfindungsgemäße Regelung bezüglich der Einstellung der unerwünschten Schwersieder- und Leichtsiederkomponenten im Seitenprodukt (Mittelsiederfraktion) beschrieben.
(e) Das Aufteilungsverhältnis des ablaufenden Flüssigkeitsstromes am oberen Ende des oberen Zulaufteils (2) zum ablaufenden Flüssigkeitsstrom am oberen Ende des oberen Entnahmeteils (3) wird bevorzugt derart eingestellt, daß die Konzentration einer Schwersiederkomponente, für die im Flüssigkeitsstrom der Seitenentnahmestelle (Seitenprodukt) ein bestimmter Grenzwert erzielt werden soll, am unteren Ende des Kopfteils (1) in der Flüssig und/oder Gasphase, bevorzugt in der Flüssigphase, 10 bis 80 %; bevorzugt 30 bis 50 % dieses Grenzwertes beträgt,
   * bei einer Erhöhung der Konzentration der Schwersiederkomponente am unteren Ende des Kopf teils (1), für die im Flüssigkeitsstrom der Seitenentnahmestelle (Seitenprodukt) ein bestimmter Grenzwert erzielt werden soll, das Aufteilungsverhältnis bezüglich des oberen Zulaufteils (2) erhöht wird, und
   * bei einer Erniedrigung der Konzentration der Schwersiederkomponente am unteren Ende des Kopf teils (1), für die im Flüssigkeitsstrom der Seitenentnahmestelle (Seitenprodukt) ein bestimmter Grenzwert erzielt werden soll, das Aufteilungsverhältnis bezüglich des oberen Zulaufteils (2) erniedrigt wird.

   Diese Maßnahme stellt auch bei Schwankungen in der Qualität und Zusammensetzung des Kolonnenzulaufs oder bei einem nicht stabilen Betrieb der Destillationskolonne die hohe Reinheit des Wertprodukts im Seitenprodukt bezüglich der unerwünschten Schwersiederkomponente sicher.
   Sind mehr als eine unerwünschte Schwersiederkomponente zugegen, so ist für die Regelung die Schwersiederkomponente maßgebend, welche die Einhaltung des Grenzwerts im Seitenprodukt am ehesten gefährdet. Steigt die Konzentration dieser Schwersiederkomponente am unteren Ende des Kopfteils (1) über die jeweilige maximalzulässige Konzentration an, ist das Aufteilungsverhältnis bezüglich des oberen Zulauf teils (2) zu erhöhen. Sinkt die Konzentration dieser Schwersiederkomponente am unteren Ende des Kopf teils (1), kann das Aufteilungsverhältnis bezüglich des oberen Zulauf teils (2) in der Regel erniedrigt werden. Allerdings nur in einem Ausmaß, bei dem diese und alle weiteren unerwünschten Schwersiederkomponenten die jeweilige maximalzulässige Konzentration am unteren Ende des Kopf teils (1) nicht überschreiten und somit die Einhaltung der Grenzwerte im Seitenprodukt gewährleisten.
   Die Bestimmung der Konzentrationen der Schwersiederkomponenten (Analyse) kann kontinuierlich oder in zeitlichen Abständen erfolgen. Sie kann sowohl innerhalb als auch außerhalb der Destillationskolonne erfolgen. Erfolgt sie außerhalb der Destillationskolonne, ist eine gasförmige und/oder flüssige Probe kontinuierlich oder in zeitlichen Abständen zu entnehmen. Wurde die Proben durch die Analyse nicht verbraucht oder verändert, oder mehr Probe genommen als benötigt, ist es gegebenenfalls möglich, die Probe wieder in die Destillationskolonne rückzuführen. Als geeignete Analysenmethoden seien beispielhaft genannt chromatografische Methoden (z.B. Gaschromatographie) oder optische Methoden (z.B. Infrarotspektroskopie). Vorteilhafterweise können die Analysenmethoden in den automatischen Regelkreis eingebunden werden, so daß das Meßergebnis als Stellgröße für die Flüssigkeitsaufteilung eingesetzt werden kann.
   Beim Einsatz des bevorzugten Gemisches, welches aus der Hydrierung von Maleinsäureanhydrid, Maleinsäure, oder deren Ester oder Halbester stammt, seien als typische Schwersiederkomponenten im Sinne der beschriebenen Regelung in (K2) beispielsweise 1,4-Butandiol und in (K3) beispielsweise Dibutylenglykol (4,4'-Dihydroxydibutylether) genannt.
   Eine nicht limitierende, schematische Darstellung zur Einbindung und Regelung ist in Fig. 7 gegeben.
(f) Die Heizleistung des Verdampfers am Sumpf teil (6) wird bevorzugt derart eingestellt, daß die Konzentration einer Leichtsiederkomponente, für die im Flüssigkeitsstrom der Seitenentnahmestelle (Seitenprodukt) ein bestimmter Grenzwert erzielt werden soll, am oberen Ende des Sumpfteils (6) in der Flüssig und/oder Gasphase, bevorzugt in der Flüssigphase, 10 bis 80 %; bevorzugt 30 bis 50 % dieses Grenzwertes beträgt,
   * bei einer Erhöhung der Konzentration der Leichtsiederkomponenten am oberen Ende des Sumpfteils (6), für die im Flüssigkeitsstrom der Seitenentnahmestelle (Seitenprodukt) ein bestimmter Grenzwert erzielt werden soll, die Heizleistung des Verdampfers am Sumpf teil (6) erhöht wird, und
   * bei einer Erniedrigung der Konzentration der Leichtsiederkomponenten am oberen Ende des Sumpfteils (6), für die im Flüssigkeitsstrom der Seitenentnahmestelle (Seitenprodukt) ein bestimmter Grenzwert erzielt werden soll, die Heizleistung des Verdampfers am Sumpf teil (6) verringert wird.

   Diese Maßnahme stellt auch bei Schwankungen in der Qualität und Zusammensetzung des Kolonnenzulaufs oder bei einem nicht stabilen Betrieb der Destillationskolonne die hohe Reinheit des Wertprodukts im Seitenprodukt bezüglich der unerwünschten Leichtsiederkomponente sicher.
   Sind mehr als eine unerwünschte Leichtsiederkomponente zugegen, so ist für die Regelung die Leichtsiederkomponente maßgebend, welche die Einhaltung des Grenzwerts im Seitenprodukt am ehesten gefährdet. Steigt die Konzentration dieser Leichtsiederkomponente am oberen Ende des Sumpfteils (6) an, ist die Heizleistung des Verdampfers am Sumpfteil (6) zu erhöhen. Sinkt die Konzentration dieser Leichtsiederkomponente am oberen Ende des Sumpf teils (6), kann die Heizleistung des Verdampfers am Sumpfteil (6) in der Regel erniedrigt werden. Allerdings nur in einem Ausmaß, bei dem diese und alle weiteren unerwünschten Leichtsiederkomponenten die jeweilige maximalzulässige Konzentration am oberen Ende des Sumpfteils (6) nicht überschreiten und somit die Einhaltung der Grenzwerte im Seitenprodukt gewährleisten.
   Für die Bestimmung der Konzentrationen der Leichtsiederkomponenten gelten prinzipiell die entsprechenden Ausführungen in Punkt (e), welche auch an dieser Stelle als eingebunden betrachtet werden.
   Beim Einsatz des bevorzugten Gemisches, welches aus der Hydrierung von Maleinsäureanhydrid, Maleinsäure, oder deren Ester oder Halbester stammt, seien als typische Leichtsiederkomponenten im Sinne der beschriebenen Regelung in (K2) beispielsweise Wasser und in (K3) beispielsweise γ-Butyrolacton genannt.
   Eine nicht limitierende, schematische Darstellung zur Einbindung und Regelung ist in Fig. 7 gegeben.
(g) Die Entnahme des Kopfprodukts wird bevorzugt temperaturgeregelt durchgeführt, wobei zur Regelung der Temperatur eine Meßstelle im Kopfteil (1) verwendet wird. Die verwendete Temperaturmeßstelle befindet sich bevorzugt um 3 bis 8, besonders bevorzugt um 4 bis 6 theoretische Trennstufen unterhalb des oberen Endes des Kopfteils (1).
   Die direkte Stellgröße zur geregelten Entnahme des Kopfprodukts kann dabei die Ablaufmenge des Kopfprodukts, das Rücklaufverhältnis oder bevorzugt die Rücklaufmenge sein.
   Eine nicht limitierende, schematische Darstellung zur Einbindung und Regelung ist in Fig. 7 gegeben.
(h) Die Entnahme des Sumpfprodukts wird bevorzugt temperaturgeregelt durchgeführt, wobei zur Regelung der Temperatur eine Meßstelle im Sumpfteil (6) verwendet wird. Die verwendete Temperaturmeßstelle befindet sich bevorzugt um 3 bis 8, besonders bevorzugt um 4 bis 6 theoretische Trennstufen oberhalb des unteren Endes des Sumpfteils (6).
   Die direkte Stellgröße zur geregelten Entnahme des Sumpfprodukts kann dabei die Ablaufmenge des Sumpfprodukts sein.
   Eine nicht limitierende, schematische Darstellung zur Einbindung und Regelung ist in Fig. 7 gegeben.
(i) Die Entnahme des Flüssigkeitsstrom an der Seitenentnahmestelle (Seitenprodukt) wird bevorzugt standgeregelt durchführt, wobei als Regelgröße der Flüssigkeitsstand im Verdampfer am Sumpf teil (6) verwendet wird.

Eine nicht limitierende, schematische Darstellung zur Einbindung und Regelung ist in Fig. 7 gegeben.

Wie eingangs erwähnt ist das erfindungsgemäße Regelungskonzept durch mindestens einem, bevorzugt mehrerer der Merkmale (a) bis (i) charakterisiert. Besonders bevorzugt ist die Kombination aller Merkmale (a) bis (i).

Fig. 7 zeigt schematisch die Einbindung der verschiedenen Apparate und Meßstellen in eine Trennwandkolonne und deren meß - und regeltechnische Verschaltung. Die Nomenklatur der Meß- und Regeleinrichtungen wurde nach den üblichen Konventionen vorgenommen. Es bedeuten:
- L: Flüssigkeitsstand
- Q: Analysenwert (Konzentration einer Komponente i oder alternativ integrale Meßgröße, z.B. Brechungsindex oder Temperaturwert)
- T: Temperatur.

"C" steht für Regelung, d.h. über einen entsprechenden Regler wird ein Steuersignal ausgegeben (gestrichelte Linie) .

Gegenüber den vorbeschriebenen Regelungskonzepten ermöglicht das erfindungsgemäße Regelungskonzept die Erzielung einer hohen Reinheit der Wertprodukte an den Seitenentnahmestellen und eine flexible und zugleich einfache Prozeßführung, welche auch bei Schwankungen in der Menge der Produktströme und deren Zusammensetzung einen sicheren Betrieb des Verfahrens unter Einhaltung der hohen Reinheitsanforderungen und eines geringen Energieverbrauchs gewährleistet.

Besonders bevorzugt beim erfindungsgemäßen Verfahren ist die destillative Trennung in drei Destillationsblöcken, welche vier Trennwandkolonnen oder entsprechende Anordnungen von thermisch gekoppelten konventionellen Destillationskolonnen (K1a), (K1b), (K2) und (K3) umfassen, wobei in Destillationsblock
(B1) Tetrahydrofuran abgetrennt wird,
   indem man in (K1a) eine Trennung in mindestens drei Fraktionen durchführt, wobei die mit Tetrahydrofuran angereicherte Mittelsiederfraktion (K1b) zuführt und darin eine Trennung in mindestens drei Fraktionen durchführt, wobei als Mittelsiederfraktion Tetrahydrofuran hoher Reinheit gewonnen werden kann und die Leichtsiederfraktion zu (K1a) rückgeführt wird, und die aus (K1a) stammende Schwersiederfraktion dem Destillationsblock (B2) zugeführt wird, wobei in Destillationsblock
(B2) γ-Butyrolacton abgetrennt wird,
   indem man in (K2) eine Trennung in mindestens drei Fraktionen durchführt, wobei als Mittelsiederfraktion γ-Butyrolacton hoher Reinheit gewonnen werden kann und die Schwersiederfraktion dem Destillationsblock (B3) zugeführt wird, wobei in Destillationsblock
(B3) 1,4-Butandiol abgetrennt wird,
   indem man in (K3) eine Trennung in mindestens drei Fraktionen durchführt, wobei als Mittelsiederfraktion 1,4-Butandiol hoher Reinheit gewonnen werden kann.

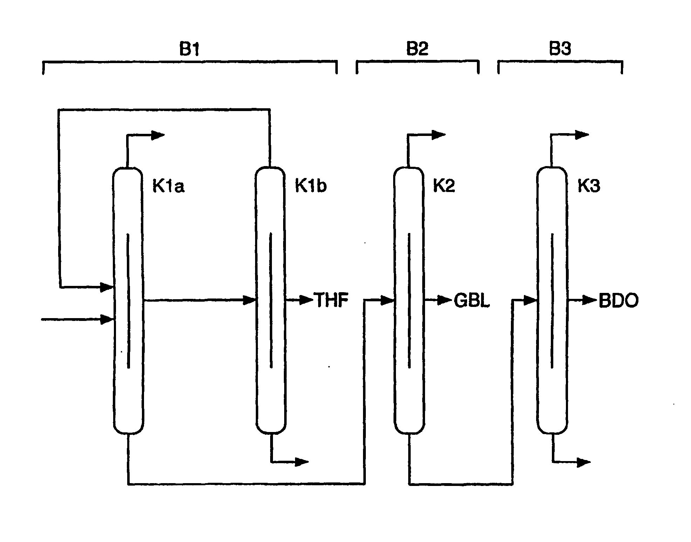

Eine schematische Darstellung dieser Anordnung ist in Fig. 8 gegeben. Die drei Wertprodukte wurden dabei wie folgt abgekürzt

| | |
|---|---|
| (THF) | Tetrahydrofuran hoher Reinheit. |
| (GBL) | γ-Butyrolacton hoher Reinheit. |
| (BDO) | 1,4-Butandiol hoher Reinheit. |

Es sei explizit darauf hingewiesen, daß die Anordnung außer den vier Trennwandkolonnen oder den entsprechenden Anordnungen thermisch gekoppelter konventioneller Destillationskolonnen noch weitere Apparate, wie beispielsweise weitere Destillationskolonnen, Pumpen, Wärmetauscher oder Behälter enthalten kann.

Des weiteren sind beim erfindungsgemäßen Verfahren auch besonders bevorzugt Anordnungen, welche sich aus der zuvor beschriebenen Anordnung der drei Destillationsblöcke (B1-B2-B3) durch Wegfall eines oder zweier Destillationsblöcke ergeben. Konkret sind damit die Kombinationen (B1), (B2), (B3), (B1-B2), (B1-B3) und (B2-B3) gemeint. In diesen Fällen werden nicht alle drei Wertprodukte Tetrahydrofuran, γ-Butyrolacton und 1,4-Butandiol isoliert, sondern nur zwei oder lediglich eines davon.

In einer bevorzugten Ausführungsform wird eine Verschaltung eingesetzt, welche die drei Destillationsblöcke
(B1) Destillationsblock zur Abtrennung von Tetrahydrofuran, welcher in der Trennwandkolonne oder der entsprechenden Anordnung von thermisch gekoppelten konventionellen Destillationskolonnen (K1a) eine Trennung in mindestens drei Fraktionen ermöglicht, wobei die mit Tetrahydrofuran angereicherte Mittelsiederfraktion der Trennwandkolonnen oder der entsprechenden Anordnung von thermisch gekoppelten konventionellen Destillationskolonnen (K1b) zugeführt wird, welche eine Trennung in mindestens drei Fraktionen ermöglicht, wobei als Mittelsiederfraktion Tetrahydrofuran hoher Reinheit gewonnen werden kann und die Leichtsiederfraktion zu (K1a) rückgeführt werden kann;
(B2) Destillationsblock zur Abtrennung von γ-Butyrolacton, welcher in der Trennwandkolonnen oder der entsprechenden Anordnung von thermisch gekoppelten konventionellen Destillationskolonnen (K2) eine Trennung in mindestens drei Fraktionen ermöglicht, wobei als Mittelsiederfraktion γ-Butyrolacton hoher Reinheit gewonnen werden kann;
(B3) Destillationsblock zur Abtrennung von 1,4-Butandiol, welcher in der Trennwandkolonnen oder der entsprechenden Anordnung von thermisch gekoppelten konventionellen Destillationskolonnen (K3) eine Trennung in mindestens drei Fraktionen ermöglicht, wobei als Mittelsiederfraktion 1,4-Butandiol hoher Reinheit gewonnen werden kann;
in der Reihenfolge (B1) vor (B2) vor (B3) umfasst. Eine schematische Darstellung dieser Anordnung ist in Fig. 8 gegeben. Die Kolonnen sind wie zuvor beschrieben ausgestaltet und bevorzugt mit geordneten Packungen bestückt. Die Kolonnen werden bevorzugt im Bereich der zuvor genannten Betriebsparameter betrieben.

Ein Gemisch, welches aus der Hydrierung von Maleinsäureanhydrid, Maleinsäure, deren Ester oder Halbester stammt und die Wertprodukte Tetrahydrofuran, γ-Butyrolacton und 1,4-Butandiol sowie Wasser und verschiedene Nebenprodukte enthält, wird kontinuierlich in die seitliche Zulaufstelle von (K1a) geleitet. Über Kopf werden die Leichtsieder, beispielsweise Butadien, 2,3-Dihydrofuran oder Methacrolein, entnommen und aus der Anlage ausgeschleust. Die Mittelsiederfraktion, welche mit Tetrahydrofuran angereichert ist, wird (K1b) zugeführt. Tetrahydrofuran hoher Reinheit wird als Mittelsiederfraktion gewonnen. Die Leichtsiederfraktion von (K1b), welche unter anderem Wasser und nicht abgetrenntes Tetrahydrofuran enthält, wird zu (K1a) rückgeführt. Die Schwersiederfraktion von (K1b) wird aus der Anlage ausgeschleust. Die Schwersiederfraktion von (K1a), welche unter anderem die Wertprodukte γ-Butyrolacton und 1,4-Butandiol enthält, wird (K2) zugeführt.

Über Kopf von (K2) werden die Leichtsieder, beispielsweise Wasser oder Methanol, entnommen und aus der Anlage ausgeschleust. γ-Butyrolacton hoher Reinheit wird als Mittelsiederfraktion gewonnen. Die Schwersiederfraktion von (K2), welche unter anderem das Wertprodukt 1,4-Butandiol enthält, wird (K3) zugeführt.

Über Kopf von (K3) werden die Leichtsieder, beispielsweise Acetale (z.B. 4-Tetrahydrofuran-2-yloxy-butanol), entnommen und aus der Anlage ausgeschleust. 1,4-Butandiol hoher Reinheit wird als Mittelsiederfraktion gewonnen. Die Schwersiederfraktion von (K3), welche beispielsweise Dibutylenglykol enthält, aus der Anlage ausgeschleust.

Die Regelung der Kolonnen erfolgt bevorzugt nach dem zuvor beschriebenen Regelkonzept.

Das erfindungsgemäße Verfahren ermöglicht die kontinuierliche Darstellung von Tetrahydrofuran, γ-Butyrolacton und/oder 1,4-Butandiol in hoher Reinheit durch destillative Trennung von Gemischen, die bei der Hydrierung von Maleinsäureanhydrid, Maleinsäure, deren Ester oder Halbester anfallen, bei gleichzeitig geringem Energieverbrauch und geringen Investitionskosten der Anlage. Des weiteren ermöglicht das erfindungsgemäße Verfahren eine flexible und zugleich einfache Prozeßführung, welche auch bei Schwankungen in der Menge der Produktströme und deren Zusammensetzung einen sicheren Betrieb des Verfahrens unter Einhaltung der hohen Reinheitsanforderungen und eines geringen Energieverbrauchs gewährleistet.

## Patentansprüche

1. Verfahren zur kontinuierlich betriebenen, destillativen Trennung von Tetrahydrofuran, γ-Butyrolacton und/oder 1,4-Butandiol enthaltenden Gemischen in mindestens drei Fraktionen, **dadurch gekennzeichnet, daß** man die Trennung in einer Anordnung von Destillationskolonnen, die mindestens eine Trennwandkolonne oder mindestens eine Anordnung von thermisch gekoppelten konventionellen Destillationskolonnen enthält, durchführt.

2. Verfahren nach Anspruch 1, **dadurch gekennzeichnet, daß** die Tetrahydrofuran, γ-Butyrolacton und/oder 1,4-Butandiol enthaltenden Gemische aus der Hydrierung von Maleinsäureanhydrid, Maleinsäure, deren Ester oder Halbester anfallen.

3. Verfahren nach den Ansprüchen 1 bis 2, **dadurch gekennzeichnet, daß** man die destillative Trennung in Destillationsblökken, welche jeweils mindestens eine Destillationskolonne enthalten, durchführt, wobei das Verfahren mindestens einen Destillationsblock aus der Reihe
(B1) Destillationsblock zur Abtrennung von Tetrahydrofuran
(B2) Destillationsblock zur Abtrennung von γ-Butyrolacton
(B3)Destillationsblock zur Abtrennung von 1,4-Butandiol
umfaßt und die Wertprodukte entsprechend ihrer Siedepunkte in der Reihenfolge (B1) vor (B2) vor (B3) gewinnt.

4. Verfahren nach Anspruch 3, **dadurch gekennzeichnet, daß** der Destillationsblock (B1) zur Abtrennung von Tetrahydrofuran mindestens zwei Destillationskolonnen umfasst, wobei der Druck in der Destillationskolonne, in der die Abtrennung des Tetrahydrofuran-Reinprodukts erfolgt, höher ist als in allen anderen Destillationskolonnen von (B1).

5. Verfahren nach Anspruch 4, **dadurch gekennzeichnet, daß** der Destillationsblock (B1) zur Abtrennung von Tetrahydrofuran zwei Trennwandkolonnen und/oder eine entsprechende Anordnung von thermisch gekoppelten konventionellen Destillationskolonnen umfasst,
(a) in der ersten Trennwandkolonne oder der entsprechenden Anordnung von thermisch gekoppelten konventionellen Destillationskolonnen (K1a) eine Trennung in mindestens drei Fraktionen erfolgt,
(b) die erhaltene, mit Tetrahydrofuran angereicherte Mittelsiederfraktion in der zweiten Trennwandkolonne oder der entsprechenden Anordnung von thermisch gekoppelten konventionellen Destillationskolonnen (K1b) in mindestens drei Fraktionen getrennt wird, wobei als Mittelsiederfraktion Tetrahydrofuran gewonnen wird und die Leichtsiederfraktion zu (K1a) rückgeführt wird.

6. Verfahren nach Anspruch 4, **dadurch gekennzeichnet, daß** der Destillationsblock (B1) zur Abtrennung von Tetrahydrofuran eine vorgeschaltete konventionelle Destillationskolonne und eine Trennwandkolonne oder eine entsprechende Anordnung von thermisch gekoppelten konventionellen Destillationskolonnen umfasst, wobei
(a) in der ersten, konventionellen Destillationskolonne eine Trennung in mindestens zwei Fraktionen erfolgt,
(b) die erhaltene, mit Tetrahydrofuran angereicherte Kopffraktion in der Trennwandkolonne oder der entsprechenden Anordnung von thermisch gekoppelten konventionellen Destillationskolonnen (K1b) in mindestens drei Fraktionen getrennt wird, wobei als Mittelsiederfraktion Tetrahydrofuran gewonnen wird und die Leichtsiederfraktion zur konventionellen Destillationskolonne rückgeführt wird.

7. Verfahren nach Anspruch 5, **dadurch gekennzeichnet, daß** man (K1a) bei einem Absolutdruck von 0,05 bis 0,2 MPa abs betreibt und die Summe der theoretischen Trennstufen des oberen und unteren Zulaufteils (2) und (4) 80 bis 110 % der Summe der theoretischen Trennstufen des oberen und unteren Entnahmeteils (3) und (5) beträgt.

8. Verfahren nach den Ansprüchen 5 oder 6, **dadurch gekennzeichnet, daß** man (K1b) bei einem Absolutdruck von 0,3 bis 1,2 MPa abs betreibt und die Summe der theoretischen Trennstufen des oberen und unteren Zulaufteils (2) und (4) 80 bis 110 % der Summe der theoretischen Trennstufen des oberen und unteren Entnahmeteils (3) und (5) beträgt.

9. Verfahren nach Anspruch 3, **dadurch gekennzeichnet, daß** der Destillationsblock (B2) zur Abtrennung von γ-Butyrolacton eine Trennwandkolonne oder eine entsprechende Anordnung von thermisch gekoppelten konventionellen Destillationskolonnen (K2) umfasst, man (K2) bei einem Absolutdruck von 0,001 bis 0,15 MPa abs betreibt und die Summe der theoretischen Trennstufen des oberen und unteren Zulaufteils (2) und (4) 80 bis 110 % der Summe der theoretischen Trennstufen des oberen und unteren Entnahmeteils (3) und (5) beträgt.

10. Verfahren nach Anspruch 3, **dadurch gekennzeichnet, daß** der Destillationsblock (B3) zur Abtrennung von 1,4-Butandiol eine Trennwandkolonne oder eine entsprechende Anordnung von thermisch gekoppelten konventionellen Destillationskolonnen (K3) umfasst, man (K3) bei einem Absolutdruck von 0,001 bis 0,05 MPa abs betreibt und die Summe der theoretischen Trennstufen des oberen und unteren Zulaufteils (2) und (4) 80 bis 110 % der Summe der theoretischen Trennstufen des oberen und unteren Entnahmeteils (3) und (5) beträgt.

11. Verfahren nach den Ansprüchen 7, 8, 9 und/oder 10, **dadurch gekennzeichnet, daß** man Kolonneneinbauten verwendet, deren Druckverlust stetig mit zunehmender Gasbelastung um mindestens 20 % pro Erhöhung des F-Faktors um 0,5 Pa^{0,5} zunimmt.

12. Verfahren nach den Ansprüchen 9 und/oder 10, **dadurch gekennzeichnet, daß** man als Kolonneneinbauten geordneten Packungen und/oder Füllkörper verwendet, deren Druckverlust stetig mit zunehmender Gasbelastung um mindestens 20 % pro Erhöhung des F-Faktors um 0,5 Pa^{0,5} zunimmt.

13. Verfahren nach den Ansprüchen 7, 8, 9, 10, 11 und/oder 12, **dadurch gekennzeichnet, daß** das Verhältnis des ablaufenden Flüssigkeitsstromes am oberen Ende des oberen Zulauf teils (2) zum ablaufenden Flüssigkeitsstrom am oberen Ende des oberen Entnahmeteils (3) 0,1 bis 1,0 beträgt.

14. Verfahren nach Anspruch 13, **dadurch gekennzeichnet, daß** man den am Kopfteil (1) ablaufenden Flüssigkeitsstrom in einem innerhalb oder außerhalb der Kolonne angeordneten Auffangraum sammelt und durch eine Festeinstellung oder eine Regelung zwischen oberen Zulaufteil (2) und oberen Entnahmeteil (3) aufteilt.

15. Verfahren nach den Ansprüchen 13 bis 14, **dadurch gekennzeichnet, daß** das Verhältnis des Brüdenstroms am unteren Ende des unteren Zulaufteils (4) zum Brüdenstrom am unteren Ende des unteren Entnahmeteils (5) 0,8 bis 1,2 beträgt.

16. Verfahren nach den Ansprüchen 13 bis 15, **dadurch gekennzeichnet, daß** man den Flüssigkeitsstrom auf die Zulaufstelle über eine Mengenregelung derart einstellt, daß der auf den Zulaufteil (2) und (4) insgesamt aufgegebene Flüssigkeitsstrom nicht unter 30 % des Auslegungswertes sinken kann.

17. Verfahren nach den Ansprüchen 13 bis 16, **dadurch gekennzeichnet, daß** man den ablaufenden Flüssigkeitsstrom am unteren Ende des oberen Entnahmeteils (3) zwischen dem abgeführten Flüssigkeitsstrom der Entnahmestelle und dem, auf den unteren Entnahmeteil (5) aufgegebenen Flüssigkeitsstrom durch eine Regelung derart aufteilt, daß der auf dem unteren Entnahmeteil (5) aufgegebene Flüssigkeitsstrom nicht unter 30 % des Auslegungswertes sinken kann.

18. Verfahren nach den Ansprüchen 13 bis 17, **dadurch gekennzeichnet, daß** man das Aufteilungsverhältnis des ablaufenden Flüssigkeitsstromes am oberen Ende des oberen Zulaufteils (2) zum ablaufenden Flüssigkeitsstrom am oberen Ende des oberen Entnahmeteils (3) derart einstellt, daß die Konzentration einer Schwersiederkomponente, für die im Flüssigkeitsstrom der Seitenentnahmestelle (Seitenprodukt) ein bestimmter Grenzwert erzielt werden soll, am unteren Ende des Kopfteils (1) in der Flüssig- und/oder Gasphase 10 bis 80 % dieses Grenzwertes beträgt,
(a) bei einer Erhöhung der Konzentration der Schwersiederkomponenten am unteren Ende des Kopf teils (1) das Aufteilungsverhältnis bezüglich des oberen Zulauf teils (2) erhöht wird, und
(b) bei einer Erniedrigung der Konzentration der Schwersiederkomponenten am unteren Ende des Kopfteils (1) das Aufteilungsverhältnis bezüglich des oberen Zulaufteils (2) erniedrigt wird.

19. Verfahren nach den Ansprüchen 13 bis 18, **dadurch gekennzeichnet, daß** man die Heizleistung des Verdampfers am Sumpfteil (6) derart einstellt, daß die Konzentration einer Leichtsiederkomponente, für die im Flüssigkeitsstrom der Seitenentnahmestelle (Seitenprodukt) ein bestimmter Grenzwert erzielt werden soll, am oberen Ende des Sumpf teils (6) in der Flüssig- und/oder Gasphase 10 bis 80 % dieses Grenzwertes beträgt,
(a) bei einer Erhöhung der Konzentration der Leichtsiederkomponenten am oberen Ende des Sumpfteils (6) die Heizleistung des Verdampfers am Sumpfteil (6) erhöht wird, und
(b) bei einer Erniedrigung der Konzentration der Leichtsiederkomponenten am oberen Ende des Sumpf teils (6) die Heizleistung des Verdampfers am Sumpfteil (6) verringert wird.

20. Verfahren nach den Ansprüchen 13 bis 19, **dadurch gekennzeichnet, daß** man die Entnahme des Kopfprodukts temperaturgeregelt durchführt und zur Regelung der Temperatur eine Meßstelle im Kopfteil (1) verwendet.

21. Verfahren nach den Ansprüchen 13 bis 20, **dadurch gekennzeichnet, daß** man die Entnahme des Sumpfprodukts temperaturgeregelt durchführt und zur Regelung der Temperatur eine Meßstelle im Sumpf teil (6) verwendet.

22. Verfahren nach den Ansprüchen 13 bis 21, **dadurch gekennzeichnet, daß** man die Entnahme des Flüssigkeitsstrom an der Seitenentnahmestelle (Seitenprodukt) standgeregelt durchführt und als Regelgröße den Flüssigkeitsstand im Verdampfer am Sumpfteil (6) verwendet.

23. Verfahren nach den Ansprüchen 1 bis 5 und 7 bis 22, **dadurch gekennzeichnet, daß** man die destillative Trennung in drei Destillationsblöcken durchführt, welche vier Trennwandkolonnen oder entsprechende Anordnungen von thermisch gekoppelten konventionellen Destillationskolonnen (K1a), (K1b), (K2) und (K3) umfassen.

## Claims

1. A process for the continuous fractional distillation of mixtures comprising tetrahydrofuran, γ-butyrolactone and/or 1,4-butanediol to give at least three fractions, wherein the fractionation is carried out in an assembly of distillation columns comprising at least one dividing wall column or at least one assembly of thermally coupled conventional distillation columns.

2. A process as claimed in claim 1, wherein the mixtures comprising tetrahydrofuran, γ-butyrolactone and/or 1,4-butanediol are obtained from the hydrogenation of maleic anhydride, maleic acid, its diesters or its monoesters.

3. A process as claimed in claim 1 or 2, wherein the fractional distillation is carried out in distillation blocks which each comprise at least one distillation column and the process comprises at least one distillation block selected from the group consisting of
(B1) a distillation block for separating off tetrahydrofuran,
(B2) a distillation block for separating off γ-butyrolactone and
(B3)a distillation block for separating off 1,4-butanediol,
and the desired products are isolated in the order (B1) before (B2) before (B3), corresponding to their boiling points.

4. A process as claimed in claim 3, wherein the distillation block (B1) for separating off tetrahydrofuran comprises at least two distillation columns, with the pressure in the distillation column in which the pure tetrahydrofuran product is separated off being higher than that in all other distillation columns of (B1).

5. A process as claimed in claim 4, wherein the distillation block (B1) for separating off tetrahydrofuran comprises two dividing wall columns and/or a corresponding assembly of thermally coupled conventional distillation columns,
(a) fractionation into at least three fractions occurs in the first dividing wall column or the corresponding assembly of thermally coupled conventional distillation columns (K1a),
(b) the resulting tetrahydrofuran-enriched intermediate-boiling fraction is fractionated in the second dividing wall column or the corresponding assembly of thermally coupled conventional distillation columns (K1b) to give at least three fractions, with tetrahydrofuran being isolated as intermediate-boiling fraction and the low-boiling fraction being recirculated to (K1a).

6. A process as claimed in claim 4, wherein the distillation block (B1) for separating off tetrahydrofuran comprises an upstream conventional distillation column and a dividing wall column or a corresponding assembly of thermally coupled conventional distillation columns, and
(a) fractionation into at least two fractions occurs in the first, conventional distillation column,
(b) the resulting tetrahydrofuran-enriched top fraction is fractionated in the dividing wall column or the corresponding assembly of thermally coupled conventional distillation columns (K1b) to give at least three fractions, with tetrahydrofuran being isolated as intermediate-boiling fraction and the low-boiling fraction being recirculated to the conventional distillation column.

7. A process as claimed in claim 5, wherein (K1a) is operated at an absolute pressure of from 0.05 to 0.2 MPa and the total number of theoretical plates in the upper and lower feed section (2) and (4) is from 80 to 110% of the total number of theoretical plates in the upper and lower offtake section (3) and (5).

8. A process as claimed in claim 5 or 6, wherein (K1b) is operated at an absolute pressure of from 0.3 to 1.2 MPa and the total number of theoretical plates of the upper and lower feed section (2) and (4) is from 80 to 110% of the total number of theoretical plates of the upper and lower offtake section (3) and (5).

9. A process as claimed in claim 3, wherein the distillation block (B2) for separating off γ-butyrolactone comprises a dividing wall column or a corresponding assembly of thermally coupled conventional distillation columns (K2), (K2) is operated at an absolute pressure of from 0.001 to 0.15 MPa and the total number of theoretical plates in the upper and lower feed section (2) and (4) is from 80 to 110% of the total number of theoretical plates in the upper and lower offtake section (3) and (5).

10. A process as claimed in claim 3, wherein the distillation block (B3) for separating off 1,4-butanediol comprises a dividing wall column or a corresponding assembly of thermally coupled conventional distillation columns (K3), (K3) is operated at an absolute pressure of from 0.001 to 0.05 MPa and the total number of theoretical plates in the upper and lower feed section (2) and (4) is from 80 to 110% of the total number of theoretical plates in the upper and lower offtake section (3) and (5).

11. A process as claimed in claim 7, 8, 9 or 10, wherein use is made of column internals whose pressure drop increases continually with increasing gas throughput by at least 20% per increase of the F factor by 0.5 Pa^{0.5}.

12. A process as claimed in claim 9 or 10, wherein the column internals used are ordered packings and/or random packing elements whose pressure drop increases continually with increasing gas throughput by at least 20% per increase of the F factor by 0.5 Pa^{0.5}.

13. A process as claimed in claim 7, 8, 9, 10, 11 or 12, wherein the ratio of the downflowing liquid stream at the upper end of the upper feed section (2) to the downflowing liquid stream at the upper end of the upper offtake section (3) is from 0.1 to 1.0.

14. A process as claimed in claim 13, wherein the liquid stream flowing downward at the top section (1) is collected in a collection space located inside or outside the column and is divided between the upper feed section (2) and the upper offtake section (3) by means of a fixed setting or a regulator.

15. A process as claimed in any of claims 13 to 14, wherein the ratio of the vapor stream at the lower end of the lower feed section (4) to the vapor stream at the lower end of the lower offtake section (5) is from 0.8 to 1.2.

16. A process as claimed in any of claims 13 to 15, wherein the liquid flow to the feed point is set by means of a flow regulator so that the total liquid stream flowing into the feed section (2) and (4) cannot drop below 30% of the design value.

17. A process as claimed in any of claims 13 to 16, wherein the downflowing liquid stream at the lower end of the upper offtake section (3) is divided between the liquid stream discharged at the offtake and the liquid stream flowing into the lower offtake section (5) by means of a regulator so that the liquid stream flowing into the lower offtake section (5) cannot drop below 30% of the design value.

18. A process as claimed in any of claims 13 to 17, wherein the split ratio of the downflowing liquid stream at the upper end of the upper feed section (2) to the downflowing liquid stream at the upper end of the upper offtake section (3) is set so that the concentration of a high-boiling component for which a particular limit value is to be achieved in the liquid stream from the side offtake point (side product) is from 10 to 80% of this limit value in the liquid and/or gas phase at the lower end of the top section (1), and
(a) the split ratio is increased in favor of the upper feed section (2) when the concentration of the high-boiling component at the lower end of the top section (1) is increased, and
(b) the split ratio is decreased in respect of the upper feed section (2) when the concentration of the high-boiling component at the lower end of the top section (1) is decreased.

19. A process as claimed in any of claims 13 to 18, wherein the heating power of the vaporizer on the bottom section (6) is set so that the concentration of a low-boiling component for which a particular limit value is to be achieved in the liquid stream of the side offtake point (side product) is from 10 to 80% of this limit value in the liquid and/or gas phase at the upper end of the bottom section (6), and
(a) the heating power of the vaporizer on the bottom section (6) is increased when the concentration of the low-boiling component at the upper end of the bottom section (6) is increased, and
(b) the heating power of the vaporizer on the bottom section (6) is reduced when the concentration of the low-boiling component at the upper end of the bottom section (6) is decreased.

20. A process as claimed in any of claims 13 to 19, wherein the top product is taken off in an temperature-regulated fashion and a temperature measurement point in the top section (1) is used for regulation.

21. A process as claimed in any of claims 13 to 20, wherein the bottom product is taken off in a temperature-regulated fashion and a temperature measurement point in the bottom section (6) is used for regulation.

22. A process as claimed in any of claims 13 to 21, wherein the liquid stream taken off at the side offtake point (side product) is taken off in a level-regulated fashion and the liquid level in the vaporizer on the bottom section (6) is used as control parameter.

23. A process as claimed in any of claims 1 to 5 and 7 to 22, wherein the fractional distillation is carried out in three distillation blocks which comprise four dividing wall columns or corresponding assemblies of thermally coupled conventional distillation columns (K1a), (K1b), (K2) and (K3).

## Revendications

1. Procédé de séparation par distillation, selon une exploitation en mode continu, de mélanges contenant du tétrahydrofuranne, de la γ-butyrolactone et/ou du 1,4-butanediol en au moins trois fractions, **caractérisé en ce que** l'on effectue la séparation dans un aménagement de colonnes de distillation, qui contient au moins une colonne à paroi séparatrice ou au moins un aménagement de colonnes de distillation conventionnelles couplées thermiquement.

2. Procédé selon la revendication 1, **caractérisé en ce que** les mélanges contenant du tétrahydrofuranne, de la γ-butyrolactone et/ou du 1,4-butanediol sont obtenus par hydrogénation de l'anhydride d'acide maléique, de l'acide maléique, de leurs esters ou de leurs semi-esters.

3. Procédé selon les revendications 1 et 2, **caractérisé en ce que** l'on effectue la séparation par distillation dans des blocs de distillation qui contiennent, respectivement, au moins une colonne de distillation, le procédé comprenant au moins un bloc de distillation de la série constituée :
(B1) d'un bloc de distillation pour séparer le tétrahydrofuranne,
(B2) d'un bloc de distillation pour séparer la γ-butyrolactone
(B3) d'un bloc de distillation pour séparer le 1,4-butanediol
et **en ce que** l'on obtient les produits intéressants en fonction de leurs points d'ébullition dans l'ordre (B1) précédant (B2) précédant (B3).

4. Procédé selon la revendication 3, **caractérisé en ce que** le bloc de distillation (B1), destiné à la séparation du tétrahydrofuranne, comprend au moins deux colonnes de distillation, la pression exercée dans la colonne de distillation où se fait la séparation du produit pur de tétrahydrofuranne étant plus élevée que dans toutes les autres colonnes de distillation de (B1).

5. Procédé selon la revendication 4, **caractérisé en ce que** le bloc de distillation (B1), destiné à la séparation du tétrahydrofuranne, comprend deux colonnes à paroi séparatrice et/ou un aménagement correspondant de colonnes de distillation conventionnelles couplées thermiquement, de sorte que :
(a) dans la première colonne à paroi séparatrice ou dans l'aménagement correspondant de colonnes de distillation conventionnelles couplées thermiquement (K1a), il se produise une séparation en au moins trois fractions, et que
(b) la fraction à point d'ébullition moyen obtenue, enrichie en tétrahydrofuranne, soit séparée dans la deuxième colonne à paroi séparatrice ou dans l'aménagement correspondant de colonnes de distillation conventionnelles couplées thermiquement (K1b), en au moins trois fractions, le tétrahydrofuranne étant recueilli comme fraction à point d'ébullition moyen et la fraction à faible point d'ébullition étant reconduite au système (K1a).

6. Procédé selon la revendication 4, **caractérisé en ce que** le bloc de distillation (B1), destiné à la séparation du tétrahydrofuranne, comprend une colonne de distillation conventionnelle montée en amont et une colonne à paroi séparatrice ou un aménagement correspondant de colonnes de distillation conventionnelles couplées thermiquement, de sorte que :
(a) dans la première colonne de distillation conventionnelle, il se produise une séparation en au moins deux fractions, et que
(b) la fraction de tête obtenue, enrichie en tétrahydrofuranne, soit séparée dans la colonne à paroi séparatrice ou dans l'aménagement correspondant de colonnes de distillation conventionnelles couplées thermiquement (K1b), en au moins trois fractions, le tétrahydrofuranne étant recueilli comme fraction à point d'ébullition moyen et la fraction à faible point d'ébullition étant reconduite à la colonne de distillation conventionnelle.

7. Procédé selon la revendication 5, **caractérisé en ce que** l'on exploite le système (K1a) à une pression absolue de 0,05 à 0,2 MPa et **en ce que** la somme des étages de séparation théoriques des parties d'alimentation supérieure et inférieure (2) et (4) représente 80 à 110 % de la somme des étages de séparation théoriques des parties de soutirage supérieure et inférieure (3) et (5).

8. Procédé selon les revendications 5 ou 6, **caractérisé en ce que** l'on exploite le système (K1b) à une pression absolue de 0,3 à 1,2 MPa et **en ce que** la somme des étages de séparation théoriques des parties d'alimentation supérieure et inférieure (2) et (4) représente 80 à 110 % de la somme des étages de séparation théoriques des parties de soutirage supérieure et inférieure (3) et (5).

9. Procédé selon la revendication 3, **caractérisé en ce que** le bloc de distillation (B2), destiné à la séparation de la γ-butyrolactone, comprend une colonne à paroi séparatrice ou un aménagement correspondant de colonnes de distillation conventionnelles couplées thermiquement (K2), **en ce que** l'on exploite l'aménagement (K2) à une pression absolue de 0,001 à 0,15 MPa et **en ce que** la somme des étages de séparation théoriques des parties d'alimentation supérieure et inférieure (2) et (4) représente 80 à 110 % de la somme des étages de séparation théoriques des parties de soutirage supérieure et inférieure (3) et (5).

10. Procédé selon la revendication 3, **caractérisé en ce que** le bloc de distillation (B3), destiné à la séparation du 1,4-butanediol, comprend une colonne à paroi séparatrice ou un aménagement correspondant de colonnes de distillation conventionnelles couplées thermiquement (K3), **en ce que** l'on exploite l'aménagement (K3) à une pression absolue de 0,001 à 0,05 MPa et **en ce que** la somme des étages de séparation théoriques des parties d'alimentation supérieure et inférieure (2) et (4) représente 80 à 110 % de la somme des étages de séparation théoriques des parties de soutirage supérieure et inférieure (3) et (5).

11. Procédé selon les revendications 7, 8, 9 et/ou 10, **caractérisé en ce que** l'on utilise des éléments encastrés de colonne, dont la perte de pression s'intensifie constamment à mesure qu'augmente la charge de gaz, à raison d'au moins environ 20 % par augmentation du facteur F d'environ 0,5 Pa^{0,5}.

12. Procédé selon les revendications 9 et/ou 10, **caractérisé en ce que** l'on utilise, comme éléments encastrés de colonne, des garnissages ordonnés et/ou des corps de garnissage, dont la perte de pression s'intensifie constamment à mesure qu'augmente la charge de gaz, à raison d'au moins environ 20 % par augmentation du facteur F d'environ 0,5 Pa^{0,5}.

13. Procédé selon les revendications 7, 8, 9, 10, 11 et/ou 12, **caractérisé en ce que** le rapport du courant de liquide s'écoulant à l'extrémité supérieure de la partie d'alimentation supérieure (2) au courant de liquide s'écoulant à l'extrémité supérieure de la partie de soutirage supérieure (3) est de 0,1 à 1,0.

14. Procédé selon la revendication 13, **caractérisé en ce que** l'on collecte le courant de liquide s'écoulant dans la partie de tête (1) dans un espace collecteur aménagé à l'intérieur ou à l'extérieur de la colonne, et **en ce qu'**on le répartit, par le biais d'un réglage fixe ou d'une régulation, en une partie d'alimentation supérieure (2) et en une partie de soutirage supérieure (3).

15. Procédé selon les revendications 13 et 14, **caractérisé en ce que** le rapport du courant de fumées à l'extrémité inférieure de la partie d'alimentation inférieure (4) au courant de fumées à l'extrémité inférieure de la partie de soutirage inférieure (5) est de 0,8 à 1,2.

16. Procédé selon les revendications 13 à 15, **caractérisé en ce que** l'on règle le courant de liquide au niveau du site d'alimentation via un réglage de quantité, de manière à ce que le courant de liquide délivré en tout aux parties d'alimentation (2) et (4) ne puisse descendre en dessous de 30 % de la valeur fixée.

17. Procédé selon les revendications 13 à 16, **caractérisé en ce que** l'on répartit le courant de liquide s'écoulant à l'extrémité inférieure de la partie de soutirage supérieure (3) en courant de liquide évacué du site de soutirage et en courant de liquide délivré à la partie de soutirage inférieure (5), par le biais d'un réglage qui permette au courant de liquide délivré à la partie de soutirage inférieure (5) de ne pas descendre en dessous de 30 % de la valeur fixée.

18. Procédé selon les revendications 13 à 17, **caractérisé en ce que** l'on règle le rapport de séparation du courant de liquide s'écoulant à l'extrémité supérieure de la partie d'alimentation supérieure (2) au courant de liquide s'écoulant à l'extrémité supérieure de la partie de soutirage supérieure (3), de manière à ce que la concentration d'un composant à point d'ébullition élevé, pour laquelle on doit atteindre une certaine valeur limite dans le courant de liquide du site de soutirage latéral (produit latéral), s'élève, à l'extrémité inférieure de la partie de tête (1) dans la phase liquide et/ou gazeuse, à une valeur de 10 à 80 % de la valeur limite en question, de sorte que :
(a) dans le cas d'une augmentation de la concentration des composants à point d'ébullition élevé à l'extrémité inférieure de la partie de tête (1), on augmente le rapport de séparation, sur la base de la partie d'alimentation supérieure (2), et que
(b) dans le cas d'une baisse de la concentration des composants à point d'ébullition élevé à l'extrémité inférieure de la partie de tête (1), on diminue le rapport de séparation, sur la base de la partie d'alimentation supérieure (2).

19. Procédé selon les revendications 13 à 18, **caractérisé en ce que** l'on règle la puissance de chauffage de l'évaporateur sur la partie de bas de colonne (6) de manière à ce que la concentration d'un composant à faible point d'ébullition, pour laquelle on doit atteindre une certaine valeur limite dans le courant de liquide du site de soutirage latéral (produit latéral), s'élève, à l'extrémité supérieure de la partie de bas de colonne (6) dans la phase liquide et/ou gazeuse, à une valeur de 10 à 80 % de la valeur limite en question, de sorte que :
(a) dans le cas d'une augmentation de la concentration des composants à faible point d'ébullition à l'extrémité supérieure de la partie de bas de colonne (6), on augmente la puissance de chauffage de l'évaporateur dans la partie de bas de colonne (6), et que
(b) dans le cas d'une baisse de la concentration des composants à faible point d'ébullition à l'extrémité supérieure de la partie de bas de colonne (6), on diminue la puissance de chauffage de l'évaporateur sur la partie de bas de colonne (6).

20. Procédé selon les revendications 13 à 19, **caractérisé en ce que** l'on effectue le soutirage du produit de tête à température réglée, et **en ce que** l'on utilise une position de mesure dans la partie de tête (1) pour régler la température.

21. Procédé selon les revendications 13 à 20, **caractérisé en ce que** l'on effectue le soutirage du produit de bas de colonne à température réglée, et **en ce que** l'on utilise une position de mesure dans la partie de bas de colonne (6) pour régler la température.

22. Procédé selon les revendications 13 à 21, **caractérisé en ce que** l'on effectue le soutirage du courant de liquide au niveau du site de soutirage latéral (produit latéral) à niveau réglé et **en ce que** l'on utilise comme valeur de réglage le niveau de liquide dans l'évaporateur sur la partie de bas de colonne (6).

23. Procédé selon les revendications 1 à 5 et 7 à 22, **caractérisé en ce que** l'on effectue la séparation par distillation dans trois blocs de distillation, qui comprennent quatre colonnes à paroi séparatrice ou les aménagements correspondants de colonnes de distillation conventionnelles couplées thermiquement (K1a), (K1b), (K2) et (K3).
